# EUROPEAN PATENT SPECIFICATION

(11) **EP 1 286 689 B1**
(45) Date of publication and mention of the grant of the patent: **30.09.2009**
(21) Application number: 01960261.4
(22) Date of filing: 01.06.2001
(51) Int. Cl.: A61K 38/27, A61P 7/00, C12N 5/08

(54) **HUMAN GROWTH HORMONE TO STIMULATE MOBILIZATION OF PLURIPOTENT HEMATOPOIETIC STEM CELLS**
MENSCHLICHES WACHSTUMSHORMON ZUR STIMULIERUNG DER MOBILISIERUNG PLURIPOTENTER HÄMAPOIETISCHER STAMMZELLEN
HORMONE DE CROISSANCE HUMAINE POUR STIMULER LA MOBILISATION DE CELLULES SOUCHES HEMATOPOIETIQUES MULTIPOTENTES

(30) Priority: 07.06.2000 EP 00111834
(43) Date of publication of application: 05.03.2003
(73) Proprietor: Merck Serono SA, 1267 Coinsins, Vaud (CH)
(72) Inventor: GIANNI, Alessandro, Massimo, I-20135 Milan (IT)
(74) Representative: Weiss, Wolfgang
(86) International application number: PCT/EP2001/006249
(87) International publication number: WO 2001/093900

(56) References cited:
- EP-A- 1 016 413
- EP-A- 1 016 414
- PETZER ANDREAS L ET AL: "Self-renewal of primitive human hematopoietic cells (long-term-culture-initiating cells) in vitro and their expansion in defined medium." PROCEEDINGS OF THE NATIONAL ACADEMY OF SCIENCES OF THE UNITED STATES, vol. 93, no. 4, 1996, pages 1470-1474, XP002160717 1996 ISSN: 0027-8424 cited in the application
- KOTZMANN H ET AL: "The influence of growth hormone substitution therapy on erythroid and myeloid progenitor cells and on peripheral blood cells in adult patients with growth hormone deficiency." EUROPEAN JOURNAL OF CLINICAL INVESTIGATION, (1996 DEC) 26 (12) 1175-81., XP000982332 cited in the application
- TIAN Z G ET AL: "Recombinant human growth hormone promotes hematopoietic reconstitution after syngeneic bone marrow transplantation in mice" STEM CELLS,US,ALPHAMED PRESS, DAYTON, OH, vol. 16, no. 3, 1998, pages 193-199, XP002114334 ISSN: 1066-5099

## Description

### FIELD OF THE INVENTION

The invention relates to the use of growth hormone and derivatives thereof for the manufacture of a medicament to increase the number of CD34 negative pluripotent peripheral blood cells capable of regenerating hematopoiesis, in particular to increase the number of long-term culture-initiating cells (LTC-IC).

The invention further relates to the use of a combination of growth hormone and G-CSF (granulocyte-colony stimulating factor) to increase the number of CD34 negative pluripotent peripheral blood cells capable of regenerating hematopoiesis in vivo.

### BACKGROUND OF THE INVENTION

Bone marrow transplantation (BMT) is a clinical procedure in which pluripotent hematopoietic cells obtained from bone marrow are transplanted to a patient. BMT is the treatment of choice in several hematological disorders, including malignanaes, Severe Combined Immune Deficiencies (SCIDs), congenitally or genetically determined hematopoietic abnormalities, anemia, aplastic anemia, leukemia and osteopetrosis (Fischer et al., 1998). In the last ten years, the use of BMT grew from less than 5'000to more than 40'000 annually (Waters et al., 1998).

Primitive or pluripotent stem cells usually reside in the bone marrow, where they generate progeny pluripotent stem cells (self-renewal) or committed progenitor cells (colony-forming cells), which are determined to produce only one or a few types of blood cells. Stem cells can be roughly divided into lymphoid stem cells, giving rise to T- and B-cells, and myeloid stem cells, giving rise to different types of lymphocytes, monocytes, erythrocytes and megakaryocytes (Alberts et al., "Molecular Biology of the Cell", 3rd edition 1994).

Primitive hematopoietic progenitor cells usually express on their surface the CD34 antigen, which is the "classical" marker for primitive progenitor cells having the above-outlined potential of self-renewal and the ability to generate colony forming cells as well as blood cells (see, e.g. Haas and Murea, 1995 or Lemoli et al, 1998).

Under steady state conditions, the majority of hematopoietic stem and progenitor cells reside in the bone marrow and only a low number of these cells are detectable in peripheral blood. However, additional stem cells can be mobilized into the peripheral blood by treatment with myelosuppressive agents and/or certain hematopoietic growth factors (Van Hoef, 1998). Studies have demonstrated that peripheral blood stem cells (PBSC) infused in a host exhibit enhanced potential for engraftment as compared to bone marrow-derived stem and progenitor cells (Gianni et al., 1989; Larsson et al., 1998). Thus, PBSC mobilized by chemotherapy, hematopoietic growth factors or the combination of these modalities are currently used in both autologous and non-autologous transplantation settings (Van Hoef, 1998 ; Anderlini and Korbling, 1997). In the case of non-autologous transplantation, the donors of stem cells are normal individuals and the procedure for mobilization of stem cells into the blood stream has to be achieved with minimal discomfort. In this case, stem cells mobilization with hematopoietic growth factors is preferred to the treatment with antiblastic drugs (i.e. cyclophosphamide).

Several hematopoietic growth factors, such as G-CSF, EPO and CSF have been studied as mobilizing agents and are currently used to increase the number of PBSC prior to leukapheresis (Henry, 1997; Weaver and Testa, 1998). Leukapheresis, also called apheresis, is a method by which the hematopoietic cells are retrieved from the donor. Treatments aimed at stimulating the overall hematopoiesis may be of great interest to mobilize a large set of progenitor cells and stem cells. Increased mobilization of stem cells is extremely valuable in the context of hematopoietic stem cells transplantation by reducing the number of leukaphereses required to collect sufficient amount of hematopoietic stem cells to be transplanted.

Kotzmann et al. (1996) studied the influence of growth hormone on erythroid and myeloid progenitor cells and on peripheral blood cells in hGH deficient patients in a long term hGH substitution therapy. The amount of said cells were monitored before hGH administration, and after 3, 6, 9, 12, 18 and 24 months afterhGH administration. A significant increase in erythroid and myeloid progenitor precursor cells was not observed until after 18 and 24 months of therapy. hGH had only negligible effects on peripheral blood cells over the whole range of time. These findings thus showed that hGH has no short term effect on hematopoietic progenitor cells. Further, it had to be concluded that the reported effect of growth hormone on hematopoietic progenitor cells cannot be exploited in a situation where a large number of hematopoietic progenitor cells is needed within a short period of time.

### SUMMARY OF THE INVENTION

The invention relates to a new use of growth hormone. More specifically, it relates to the use of growth hormone and derivatives thereof for the manufacture of a medicament to increase the number of CD34 negative pluripotent peripheral blood cells capable of regenerating hematopoiesis, so as to provide said CD34 negative pluripotent peripheral blood cells in an amount for autologous transplantation in a patient in need of hematopoietic transplantation, e.g. in a human being. In particular, the invention relates to the use of a combination of growth hormone and G-CSF for this purpose. The invention further relates to uses exploiting said use of growth hormone or of the combination of growth hormone and G-CSF.

### DESCRIPTION OF THE INVENTION

The first aspect of the invention relates to the use of growth hormone as a mobilizing agent to increase the number of circulating cells capable of regenerating hematopoiesis *in vivo* in an individual. The cells contemplated according to the invention are pluripotent cells circulating in the peripheral blood. The cells are CD34 negative, i.e. lack the cell surface marker which has been traditionally associated with the subset of primitive, pluripotent hematopoietic progenitor cells.

The new mobilizing agent of the invention is growth hormone and especially human growth hormone (hGH) and derivatives thereof.

Unless it is otherwise specified, the term "GH" means growth hormone or one of its derivatives within the context of the invention.

It has now been found that, by administering growth hormone and especially human growth hormone (hGH) or a derivative thereof, a mobilization of CD34 negative progenitor cells into the peripheral blood is obtained. These cells are capable of regenerating hematopoiesis in vivo, i.e. generate blood cells when re-infused or transplanted into a human being. They are also capable of generating progeny cells when put into cell culture, all or part of the progeny cells also having a hematopoietic potential. The term "pluripotent" or "primitive" CD34 negative progenitor cells as used herein is meant to encompass those CD34 negative hematopoietic stem cells having a self-renewal potential and/or the potential to generate different kinds of bone marrow cells or blood cells. Thus, these cells can be used to reconstitute, build up, supplement or contribute to the hematopoietic system in an individual ("*in vivo*"), after having been administered to the individual. Alternatively, they can be used to regenerate hematopoietic cells or blood cells in cell culture ("*in vitro*").

Growth hormone, especially human Growth Hormone (hGH) and derivatives thereof, administered alone or in combination with other factors, represents a new use to mobilize these cells in vivo from the bone marrow into the peripheral blood. From the peripheral blood, the cells can be retrieved by leukapheresis, for example, and either stored or cultured in vitro for expansion or generation of progenitor cells, which are capable of reconstituting hematopoiesis after transplantation into an individual.

When the CD34 negative progenitor cells are put into cell culture, they grow or proliferate when subjected to the right conditions, to generate more pluripotent cells or committed cells or colony-forming cells or any other cell type able to reconstitute hematopoiesis in vivo. The progeny cells can then be used for reconstituting the bone marrow cells or blood cells in a patient, like, for example, in an individual, who has undergone myeloablative therapy.

During in vitro culturing, the CD34 negative progenitor cells may turn into CD34 positive cells. The presence or absence of the CD34 antigen on the surface of cells may be analyzed using a CD34 specific antibody by methods well known by the person skilled in the art, like, for example, ELISA of FACS. It may further be analyzed by other methods well known in the art, like RT-PCR or the like.

Human Growth Hormone (hGH), also known as somatotropin is a protein hormone produced and secreted by the somatotropic cells of the anterior pituitary. hGH plays a key role in somatic growth through its effects on the metabolism of proteins, carbohydrates and lipids. In addition to its effects on somatic growth, hGH has been shown to stimulate blood cells *in vitro* (Derfalvi et al., 1998 ; Merchav et al; 1988), to increase erythrocytes and hemoglobin counts (Valerio et al. , 1997 ; Vihervuori et al. , 1996), to enhance both proliferation and Ig (immunoglobulin) production in plasma cell lines (Kimata and Yoshida, 1994) and to stimulate CD8⁺ cell counts and, to a lesser extent CD4⁺ cell counts(Geffner, 1997).

The uses of the invention using the mobilizing agent according to the invention have several advantages :

There is usually a low number of circulating cells capable of regenerating hematopoiesis. This number is considered insufficient to provide a cell dose which is suitable for a transfer of cells by single or multiple leukapheresis in a reasonable period of time. This situation is aggravated in "low mobilizers". Patients are classified as being low mobilizers if they have less that around 10 CD34+ cells per µl of blood and/or patients having less than around 3 x 10⁸ CD34+ cells per kg of bodyweight.

Uses of the invention solve this problem by a temporary peripheralization of said cells and subsets into the circulating blood leading to a significant increase of the yield of circulating cells capable of regenerating hematopoiesis in vivo in the blood, thus minimizing the number of leukaphereses needed to achieve an engraftment dose.

Other advantages of the uses of the invention include the possibility of :
a) circumventing the need of general anasthesia,
b) harvesting even if iliac bones are damaged by previous radiotherapy or infiltrated with malignant cells,
c) achieving restoration of sustained hematopoietic functions more rapidly than with BM derived progenitor cells.
d) achieving restoration of sustained hematopoietic functions more rapidly and effectively than without a pre-treatment including a method or a use of the invention.

Generally, uses of the invention are effective and safe to mobilize to peripheral blood cells capable of regenerating hematopolesis in vivo.

Uses of the invention are not toxic in view of main parameters of toxicity which are for example tumor growth, clinical and instrumental symptoms (clinical parameters, or laboratory tests for cardiac, liver and renal function).

The increased mobilization or peripheralization of circulating cells capable of regenerating hematopoiesis in vivo obtained with the uses of the invention is extremely valuable in the context of hematopoietic stem cells transplantation by reducing the number of leukaphereses required to collect a sufficient amount of hematopoietic cells to be transplanted.

Uses of the invention also lead to a reduction of the volume of blood required to be processed during the apheresis or leukapheresis procedure in order to obtain the specified target number of cells. The advantages of processing a reduced volume of blood are that the patient spends less time on the cell separating machine, that it reduces the toxicity of the procedure, particularly in terms of the volume of anticoagulant to which the patient would be exposed during the procedure, that it reduces the machine and the operator's time.

Furthermore, the transplantation of a population of blood cells enriched with cells capable of regenerating hematopoiesis in vivo, which population is obtained from the peripheral blood by the uses of the invention has the effect to enhance reconstitution of recipient's hematopoietic and immune systems following myeloablative or antiblastic therapies.

In a first aspect, the invention concerns the use of human growth hormone or one of its derivatives for the manufacture of a medicament to increase in a patient in need of hematopoietic regeneration the number of circulating CD34 negative pluripotent peripheral blood cells capable of reconstituting hematopoiesis in vivo, so as to provide said CD34 negative pluripotent peripheral blood cells in an amount sufficient for autologous transplantation.

The term "increased" or "increase" or "enriched" generally mean in the context of the invention that the "increased" or "enriched" parameter (number) has a value which is above the standard value of this parameter. The standard value of the parameter is measured in a body or in a sample of a body which has not received any mobilizing agent of cells capable of regenerating hematopoiesis in vivo.

In a preferred embodiment, the CD34 negative pluripotent cells are long-term culture-initiating cells (LTC-IC).

LTC-IC are a subset of rare, very primitive hematopoietic stem cells having the ability to produce large numbers of mature progeny over prolonged periods of time (Petzer et al., 1996, Sutherland et al., 1994). In adult hematopoietic tissues, there is usually only a small population of these totipotent cells. This very early hematopoietic cell type can be detected and quantified using the long-term culture-initiating cell (LTC-IC) assay that measures its ability to generate granulopoietic, erythropoietic, and pluripotent colony-forming cells after 5 weeks in cultures containing a feeder layer of normal human marrow adherent cells or competent murine fibroblasts (Petzer et al., 1996). The cells are therefore called LTC-IC.

The LTC-IC is capable of regenerating all of the different lymphoid and myeloid compartments following their transplantation into myeloablated recipients and can generate progeny with the same totipotent properties.

As shown in the examples below, it has now been found that an elevated number of LTC-IC can be retrieved by leukapheresis treatment using hGH or, preferably, hGH and G-CSF. At least a portion of the LTC-IC may be negative for the CD34 antigen. The possibility of retrieving LTC-IC cells represents an important progress, since these cells are capable of reconstituting hematopoiesis when transplanted into a patient who may lack a functioning hematopoietic system or whose hematopoietic system has been destroyed by radiotherapy or chemotherapy, for example. It may even be possible to culture LTC-IC over extended periods of time to generate a large number of cells and use the*in vitro* expanded cells for re-infusion into the patient.

The LTC-IC are used for transplantation, either for the same individual (autologous transplantation). Also disclosed is the use for transplantation for an individual different from the donor (heterologous/allogeneic transplantation), or for a relative , having half of the haplotype of the donor (semi-allogeneic transplantation). If the LTC-IC are to be transplanted into a recipient different from the donor, the histocompatibility antigens have to mach in order to minimize rejection reactions. However, transplantation is also possible between donors and acceptors not having matching histocompatibility antigens, provided the T-cells have been eliminated.

The conditions in which LTC-IC are cultivated can be taken from the examples below, and are described in the literature, for instance, by Sutherland et al. (1993), Lemieux et al. (1995), Sutherland et al. (1994) or Petzer et al. (1996). Usually, the LTC-IC are administered together with all the other cell types retrieved by apheresis. From this mixture, it is the primitive hematopoietic stem cells, which reconstitute the bone marrow. Therefore, the higher the number of stem cells In the mixture is the better will reconstitution of the hematopoietic system work. It is appreciated by the person skilled in the art that the LTC-IC and/or other stem cell types may also be separated from each other by methods well known in the art, like by gradient centrifugation, for example.

Preferably, the number of circulating CD34 negative pluripotent peripheral blood cells retrieved after administration of the medicament according to the invention is at least about 50, more preferably at least about 100, 500 or most preferable more than 1000 cells per milliliter of blood.

The blood containing the CD34 negative pluripotent peripheral blood cells may be collected by any method known in the art. The cells are preferably collected by leukapheresis. Leukapheresis (also called apheresis) is a procedure, in which leukocytes are removed from the withdrawn blood and the remainder of the blood is re-transfused into the donor. The leukocytes collected may be stored or directly used for transplatation into another recipient. Leukapheresis is a non-invasive method as compared to bone marrow removal. It is therefore equally suitable for obtaining cells from healthy donors as well as from patients.

Cells capable of regenerating hematopoiesis in vivo present in the isolated population of blood cells can be further purified or enriched in order to increase the concentration of said cells.

In an advantageous embodiment, the circulating CD34 negative pluripotent peripheral blood cells are stored for a later transplantation into a human being. They may also be cultured for generation of hematopoietic progeny cells. The progeny hematopoietic cells may be LTC-IC themselves or totipotent, pluripotent, committed, clonogenic or colony forming cells or any other cell type which can be generated from LTC-IC and are used for transplantation into a patient. The hematopoietic cells may comprise lymphoid, myeloid, granulopoietic or erythropoietic cells.

The transplantation of the cells as outlined above is autologous transplantation, i.e. taken from and re-infused later into the same individual. An autologous transplantation has the advantage that immune reactions or rejection of the transplanted cells are avoided, the cells being derived from the patient himself.

Also disclosed is a transplantation that is heterologous (allogeneic), i.e. taken from an individual and transplanting the cells into another individual having a compatible pattern of histocompatibility antigens. Heterologous transplantation is advantageous in a case where the patient has few own healthy hematopoietic cells, or the cells are not easily separated from cancer cells which may be present, or the patient is too weak for a removal of healthy cells.

The use according to the invention is suitable for any malignant disease for which myelotoxic chemotherapy is indicated or applied. It is especially preferred for treating a neoplastic disease, cancer, be it solid or disseminated, leukemia, lymphoma, a hematological disorder, malignancies, congenitally or genetically determined hematopoietic abnormalities, anemia, aplastic anemia, neutropenia and/or osteopetrosis. In all of these diseases, transfer of hematopoietic cells for reconstitution of the hematopoietic system is required or desirable.

It is preferred to use the medicament according to the invention when the disease is Hodgkin's disease. Hodgkin's disease or Hodgkin's lymphoma is a lymphoma usually requiring chemotherapy. After chemotherapy, transplantation of hematopoietic cells for reconstitution or support of building up the hematopoietic system in themyeloablated patient is required.

The medicament according to the invention preferably further comprises one or several compound(s) chosen among the following groups of compounds: hematopoietic growth factors, cytokines, chemokines, monoclonal antibodies.

The growth factor group may comprise thrombopoietin (TPO).

The cytokines group may comprise IL-1, IL-3, IL-6, IL-11, Insulin-like growth factor 1 (IGF-1), G-CSF, GM-CSF or SCF for example, the chemokines group can e.g. comprise MIP-1α or MPIF-1 or -2 (myeloid progenitor inhibitory factor-1 and -2, described e.g. in the US patent 6.001,606) or the chemokine EU-2; the monoclonal antibodies may group comprise anti-VLA-4 antibodies, for example.

In a highly preferred embodiment, the medicament further comprises G-CSF. It has been surprisingly found that the administration of a combination of growth hormone and G-CSF leads to the mobilization or peripheralization of an elevated number of primitive CD34 negative cells as compared to an administration of G-CSF alone. As shown in the examples below, the amount of CD34 negative progenitor cells mobilized by GH and G-CSF may be greatly enhanced as compared to an administration of G-CSF alone.

The growth hormone may be administered in an amount of 10 to 500 µg per kg per administration, preferably in an amount of around 100 µg per kg per administration.

G-CSF may be administered in an amount of around 1 to 100 µg per kg per day, preferably in an amount of around 5 to 25 µg per kg per day. The administration of around 5 to 10 µg per day is highly preferred.

Advantageously, the administration is made by parenteral, subcutaneous, intravenous, intramuscular, intraperitoneal, transdermal or buccal routes.

The intravenous route is preferred. The administration may be daily or three times a day. A regimen, wherein the administration of growth hormone is made three times a day and the administration of G-CSF is daily, is preferred.

The administration can be made over a period of about 1 to 14 days or, until apheresis, until mobilization or peripheralization of circulating cells capable of regenerating hematopoiesis in vivo, until increase of the number of circulating cells capable of regenerating hematopoiesis in vivo or until engraftment.

In highly preferred embodiments of the invention, the administration or administrations is/are made before or after chemotherapy, radiotherapy, myelotoxic or myelosuppressive therapy, transplantation of cells capable of regenerating hematopoiesis in vivo or bone-marrow transplantation. It may be made after any of these therapies, as long as the therapy is stem cell sparing.

The administration(s) begin(s) around seven days after the beginning of a chemotherapeutic treatment or around 2 days after the end of a chemotherapeutic treatment. Any myelotoxic therapy is suitable for chemotherapy, be it a single therapeutic agent or a combination treatment, as long as it is stem cell sparing. For salvage chemotherapy, ifosphamide (IFO) and/or vinorelbine (VNR) are frequently used, for example.

In a preferred embodiment, the growth hormone used is human growth hormone, and the use of recombinant growth hormone is highly preferred. Further, the use of human, recombinant G-CSF is highly preferred.

Also disclosed is a method of preparation of a population of cells capable of regenerating hematopoiesis in a human being, comprising the steps of:
a) Administering to a donor a composition comprising growth hormone or one of its derivatives or any factor inducing growth hormone release in an amount sufficient to increase in said donor the number of circulating CD34 negative pluripotent peripheral blood cells capable of regenerating hematopoiesis In a human being; and
b) Retrieving the population of circulating CD34 negative pluripotent peripheral blood cells capable of regenerating hematopoiesis in vivo from the donor.

The method thus produces a population of CD34 negative cells capable of regenerating hematopoiesis in vivo, this population being destined for transplantation in the same or in different individuals, for example.

It is appreciated by the person skilled in the art, that the term « the administration » not only means one single administration, but also encompasses more than one, several or many administrations.

In step (b), the retrieval, collection or isolation of CD34 negative cells usually comprises isolating or removing blood or blood cells containing the pluripotent cells from a donor (a healthy or diseased individual). This may be done by any method known, the most convenient method being leukapheresis. During leukapheresis, usually fluid and red cells are re-infused into the patient, and the residual cells (the "buffy-coat") are retained.

An amount sufficient to increase the number of circulating cells capable of regenerating hematopoiesis in vivo, an amount sufficient to induce the mobilization or peripheralisation of circulating cells capable of regenerating hematopoiesis in vivo or an amount sufficient to induce mobilization of cells capable of regenerating hematopoiesis in vivo to the peripheral blood can be administered in one or several doses during one or several days.

The cells isolated from the donor may be stored, e.g. because the donor will be subjected to a specific treatment like chemotherapy, radiotherapy or any myeloablative therapy, for example. The storing of cells is usually done by freezing them in liquid nitrogen. Alternatively, they may be used for transplantation right away, e.g. given to an allogeneic recipient. Deep frozen cells may be stored for extended periods of time, like weeks, months or years. For thawing, the cells are put to 37 °C until they melt, and may then be transferred to the recipient.

Also disclosed is a method of preparation of a donor of circulating CD34 negative pluripotent peripheral blood cells, comprising administration of Growth Hormone or one of its derivatives or any factor inducing the growth hormone release in an amount sufficient to increase the number of circulating CD34 negative pluripotent peripheral blood cells.

In another aspect, a method is disclosed for increasing the number of circulating CD34 negative pluripotent peripheral blood cells in vivo in a donor by administration of a composition comprising growth homone and/or one of its derivatives and/or any factor inducing the growth hormone release to said donor.

In the methods the circulating CD34 negative pluripotent peripheral blood cells are LTC-IC.

Advantageously, the increased number of circulating CD34 negative pluripotent peripheral blood cells is at least about 50, 100, 500 or 1000 cells per milliliter of peripheral blood.

In step (b), any method to retrieve and/or isolate the CD34 negative cells may be used. The use of leukapheresis is preferred.

The specified target number of circulating cells capable of regenerating hematopoiesis in vivo is at least 2x10⁴ cells per kg of donor or recipient body weight.

The composition administered in step (a) may comprise further one or several compounds chosen among the following groups of compounds: hematopoietic growth factors, cytokines, chemokines, monoclonal antibodies. The growth factor group may comprise thrombopoietin TPO, for example. The cytokine group can comprise IL-1,IL-3, G-CSF, GM-CSF or SCF; the chemokine group comprises MIP-1α, MPIF-1, MPIF-2 or EU-2; the monoclonal antibody group comprises anti-VLA-4 antibodies.

Preferably, compositions further comprise G-CSF.

Preferably, composition comprises growth hormone and G-CSF.

In step (a), growth-hormone may be administered in an amount comprised between 10 to 500 µg/kg of body weight, in particular in an amount of about 100 µg/kg of body weight. G-CSF may be administered in an amount comprised between 1 to 100 µg/kg of body weight, in particular in an amount of around 5 to 10 µg per kilogram of body weight.

The following regimens are preferred :
- The administration of Growth Hormone is made three times a day and the administration of G-CSF is made daily;
- The administration is made by parenteral, subcutaneous, intravenous, intramuscular, intraperitoneal, transdermal or buccal routes.
- The administration is made over a period of around 14 days, until apheresis, until mobilization or peripheralization of circulating cells capable of regenerating hematopoiesis in vivo, until increase of the number of circulating cells capable of regenerating hematopoiesis In vivo or until engraftment.
- The administration is made before or after chemotherapy, radiotherapy, myelotoxic or myelosuppressive therapy, transplantation of cells capable of regenerating hematopoiesis in vivo or bone-marrow transplantation.
- The administration begins around 7 days after the beginning of a chemotherapeutic treatment or around 2 days after the end of a chemotherapeutic treatment.
- The administration is made after chemotherapeutic treatment with ifosphamide and/or vinorelbine.

Preferably, the administered growth hormone is human growth hormone, in particular recombinant growth hormone, and the administered G-CSF is human G-CSF, in particular recombinant G-CSF.
In this application :
- The term "circulating" may be replaced by the term "blood" or "peripheral blood".
- The term "preparation" in the expression "method of preparation" may be replaced by "pre-treament" or by "preparation for blood extraction or leukapheresis".
- A "donor" as recited in the methods or uses of the invention may be a human or an animal, a healthy or a sick individual (patient). Said animal is preferably a mammal and may be chosen from domestic animals such as dogs, cats etc. or animals such as pigs, horses, cattle, sheep.
- The term "hematopoiesis" can mean the formation of the blood cells.
- The term "Growth hormone" encompasses human growth hormone (hGH) and all the homologous proteins of human growth hormone of different species and all the homologs of human growth hormone in species other than human. Species other than human may be any sort of domestic animal or horse for example.

In preferred embodiments, growth hormone is human growth hormone. Human growth hormone (hGH), also known as somatropin (INN) or somatotropin is a protein hormone produced and secreted by the somatotropic cells of the anterior pituitary. hGH plays a key role in somatic growth through its effects on the metabolism of proteins, carbohydrates and lipids.

Human growth hormone is a single polypeptide chain of 101 amino acids having two disulfide bonds, one between Cys-53 and Cys-165, forming a large loop in the molecule, and the other between Cys-182 and Cys-189, forming a small loop near the C-terminus.

The term "derivative" in the expression "derivatives of growth hormone" signifies in the context of the invention, molecules which differ structurally from GH but which conserve the function of GH with respect to its direct or indirect effect on the metabolism of proteins, carbohydrates and lipids and/or its mobilization effect and/or recovery effect (i.e. "mobilization or peripheralization of circulating cells capable of regenerating hematopoiesis in vivo, increase of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo").

Derivatives of human growth hormone (hGH) included in the invention include naturally-occurring derivatives, variants and metabolic products, degradation products primarily of biosynthetic hGH and engineered derivatives of hGH produced by genetic methods. Any derivative of hGH can be used for the purpose of the present invention as long as it retains the biological activity of hGH in view of the invention.

Examples of derivatives are splice variants, oligomers, aggregates, proteolytic cleavage products, variants having substitutions, insertions or deletions of one or more amino acids etc.

Methionyl hGH is an example of derivative of hGH which is produced through recombinant DNA technology. This compound is actually a derivative of hGH having one additional methionine residue at its N-terminus (Goeddel et al., 1979).

Another example of derivative of hGH is a naturally occurring variant of hGH called 20-K-hGH which has been reported to occur in the pituitary as well as in the bloodstream (Lewis et al, 1977; 1978 and 1981). This compound, which lacks the 15 amino acid residues from Glu-32 to Gln-46, arises from an alternative splicing of the messenger ribonucleic acid (DeNoto et al., 1981).

Another example of derivative of hGH is acetylated at the N-terminus (Lewis et al., 1979).

Human growth hormone may further be in a monomeric, dimeric and higher molecular weight oligomeric form or in a mixture of said forms.

Human growth hormone may be in aggregated forms found both in the pituitary and in the circulation (Stolar et al., 1984 ; Stolar and Baumann, 1986).

The dimeric form of hGH may be of distinct types :
- a disulfide dimer connected through interchain disulfide bonds (Lewis et al., 1977),
- a covalent or irreversible dimer that is detected on sodium dodecylsulfate-polyacrylamide gels and that is not a disulfide dimer (Bewley and Li, 1975), and
- a non-covalent dimer which is easily dissociated into monomeric hGH by treatment with agents that disrupt hydrophobic interactions in proteins (Becker et al., 1987),
- a dimeric complex with Zn²⁺ (Cunningham et al., 1991).

Scatchard analysis has revealed that two Zn²⁺ ions associate per hGH dimer in a cooperative fashion, and this Zn²⁺-hGH dimeric complex was found to be more stable to denaturation than monomeric hGH (Cunningham et al., 1991).

A number of derivatives of hGH arise from proteolytic modifications of the molecule. The primary pathway for the metabolism of hGH involves proteolysis. The region of hGH around residues 130-150 is extremely susceptible to proteolysis, and several derivatives of hGH having nicks or deletions in this region have been described (Thorlacius-Ussing, 1987). This region is in the large loop of hGH, and cleavage of a peptide bond there results in the generation of two chains that are connected through the disulfide bond at Cys-53 and Cys-165. Many of these two-chain forms are reported to have increased biological activity(Singh et al., 1974).

Many derivatives of human growth hormone have been generated artificially through the use of enzymes. The enzymes trypsin and subtilisin, as well as others, have been used to modify hGH at various points throughout the molecule (Lewis et al., 1977). One such derivative, called two-chain anabolic protein (2-CAP), was formed through the controlled proteolysis of hGH using trypsin.

Another example of derivative of hGH is deamidated hGH. Asparagine and glutamine residues in proteins are susceptible to deamidation reactions under appropriate conditions. An example of deamidated hGH is pituitary hGH which has been shown to undergo this type of reaction, resulting in conversion of Asn-152 toaspartic acid and also, to a lesser extent, conversion of Gln-137 to glutamic acid (Lewis et al., 1981). Another example of deamidated hGH is biosynthetic hGH which is known to degrade under certain storage conditions, resulting in deamidation at a different asparagine (Asn-149). This is the primary site of deamidation, but deamidation at Asn-152 is also seen (Becker et al., 1988). Deamidation at Gln-137 has not been reported in biosynthetic hGH.

Another example of derivative of hGH is sulfoxide hGH. Methionine residues in proteins are susceptible to oxidation, primarily to the sulfoxide. Both pituitary-derived and biosynthetic hGH undergo sulfoxidations at Met-14 and Met-125 (Becker et al., 1988). Oxidation at Met-170 has also been reported in pituitary but not biosynthetic hGH.

Another example of derivative of hGH is truncated forms of hGH which have been produced, either through the actions of enzymes or by genetic methods. 2-CAP, generated by the controlled actions of trypsin, has the first eight residues at the N-terminus of hGH removed. Other truncated versions of hGH have been produced by modifying the gene prior to expression in a suitable host. The first 13 residues have been removed to yield a derivative having distinctive biological properties in which the polypeptide chain is not cleaved (Gertler et al., 1986).

hGH and its derivatives may be produced by recombinant DNA technology which permits production of an unlimited supply of hGH in a number of different systems. Purification of hGH or its derivatives from the culture medium is facilitated by low amounts of contaminating proteins present. In fact, it has been shown that hGH can be purified on a laboratory scale by a single purification step on a reversed-phase HPLC column.

Recombinant hGH is generally marketed as vials containing hGH plus additional excipients, e.g., glycine and mannitol, in a lyophilized form. A companion diluent vial is provided, allowing the patient to reconstitute the product to the desired concentration prior to administration of the dose.

In general, no significant differences have been observed in the pharmacokinetics or biological activities of recombinant natural sequence hGH, recombinant N-methionyl-hGH, or pituitary-derived material in humans (Moore et al., 1988 ; Jorgensen et al., 1988).

The human growth hormone as used in the present invention can include functional derivatives as noted above, as well as other types of derivatives, fragments, variants, analogs, or chemical derivatives. A functional derivative retains at least a portion of the amino acid sequence of hGH which permits its utility in accordance with the present invention; namely mobilization of circulating cells capable of regenerating hematopoiesis in vivo for example.

In the meaning of the invention, a "derivative" may be :
- A "fragment" of the human growth hormone according to the present invention refers to any subset of the molecule, that is, a shorter peptide.
- A "variant" of the human growth hormone according to the present invention refers to a molecule which is substantially similar to either the entire peptide or a fragment thereof. Variant peptides may be conveniently prepared by direct chemical synthesis of the variant peptide, using methods well known in the art.

Alternatively, amino acid variants of hGH can be prepared by mutations in the cDNA encoding the synthesized hGH derivatives. Such variants comprise deletions, insertions or substitution of residues within the amino acid sequence. Any combination of - deletions, insertions, and substitutions may also be made, provided that the final construct possesses the desired activity.

At the genetic level, these variants ordinarily are prepared by site-directed mutagenesis (as exemplified by (Adelman et al., 1983)) of nucleotides in the DNA encoding the peptide molecule, thereby producing DNA encoding the variant, and thereafter expressing the DNA in recombinant cell culture. The variants typically exhibit the same biological activity as the non-variant peptide.
- An "analog" of human growth hormone according to the present invention refers to a non-natural molecule which is substantially similar to either the entire molecule or to an active fragment thereof.
- A "chemical derivative" of human growth hormone according to the present invention contains additional chemical moieties not normally part of the human growth hormone derivative amino acid sequence. Covalent modifications of the amino acid sequence are included within the scope of this invention. Such modifications may be introduced into the human growth hormone by reacting targeted amino acid residues of the peptide with an organic derivatizing agent that is capable of reacting with selected side chains or terminal residues.

The types of substitutions which may be made in the human growth hormone according to the present invention may be based on analysis of the frequencies of amino acid changes between a homologous protein of different species. Based upon such analysis, conservative substitutions may be defined herein as exchanges within one of the following five groups:

| | |
|---|---|
| I | : Small, aliphatic, nonpolar or slightly polar residues : Ala, Ser, Thr, Pro, Gly |
| II | : Polar, negatively-charged residues and their amides : Asp, Asn, Glu, Gin |
| III | : Polar, positively-charged residues : His, Arg, Lys |
| IV | : Large, aliphatic non-polar residues : Met, Leu, Ile, Val, Cys |
| V | : Large aromatic residues : Phe, Try, Trp |

Within the foregoing groups, the following substitutions are considered to be "highly conservative":
-Asp/Glu
-His/Arg/Lys
-Phe/Tyr/Trp
-Met/Leu/Ile/Val

Semi-conservative substitutions are defined to be exchanges between two of groups (I)-(IV) above which are limited to supergroup (A), comprising (I), (II), and (III) above, or to supergroup (B), comprising (IV) and (V) above. Substitutions are not limited to the genetically encoded or even the naturally- occurring amino acids. When the epitope is prepared by peptide synthesis, the desired amino acid may be used directly. Alternatively, a genetically encoded amino acid may be modified by reacting it with an organicderivatizing agent that is capable of reacting with selected side chains or terminal residues.

Cysteinyl residues most commonly are reacted with alpha- haloacetates (and corresponding amines), such as chloroacetic acid or chloroacetamide, to give carboxylmethyl or carboxyamidomethyl derivatives. Cysteinyl residues also are derivatized by reaction with bromotrifluoroacetone, alpha-bromo-beta-(5-imidazoyl)propionic acid, chloroacetyl phosphate, N-alkylmaleimides, 3-nitro-2-pyridyl disulfide, methyl-2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Parabromophenacyl bromide is also useful ; the reaction is preferably performed in 0.1 M sodiumcacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino acid-containing residues include imidoesters such as methyl picolinimidate ; pyridoxal phosphate ; pyridoxal; chloroborohydride; trinitrobenzenesulfonic acid ; O-methyliosurea ; 2,4-pentanedione ; and transaminase-catalyzed reaction with glyoxylate.

Arginyl residues are modified by reaction with one or several conventional reagents, among them phenylglyoxal ; 2,3- butanedione ; and ninhydrin. Derivatization of arginine residues requires that the reaction be performed in alkaline conditions because of the high pKa of the guanidine functional group. Furthermore, these reagents may react with the groups of lysine, as well as the arginine epsilon-amino group.

The specific modification of tyrosyl residues per se has been studied extensively, with particular interest in introducing spectral labels into tyrosyl residues by reaction with aromatic diazonium compounds or tetranitromethane. Most commonly, N-acetylimidazole and tetranitromethane are used to form O-acetyl tyrosyl species and e-nitro derivatives, respectively.

Carboxyl side groups (aspartyl or glutamyl) are selectively modified by reaction with carbodiimides (R'N-C-N-R') such as 1-cyclohexyl-3-[2-morpholinyl-(4-ethyl)]carbodiimide or 1-ethyl-3-(4-azonia-4,4-dimethylpentyl)carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

While the present invention may be carried out with recombinant human growth hormone derivatives made by recombinant DNA technology, for instance in procaryotic or eucaryotic cells, these derivatives can also be made by conventional protein synthesis methods which are well known to those skilled in the art.

Growth hormone may be a protein, a peptide, a DNA molecule, a RNA molecule. DNA molecule and RNA molecule may encode hGH and all its derivatives including those recited above.

Growth hormone may preferably be recombinant growth hormone.

Determination of amounts of growth hormone, of one of its derivatives to be administered in a method or use of the invention described above is within the skill of the art.

Typical dosage of growth hormone, of one of its derivatives will start at about 1 microgram per kilogram of the patient or donor weight per day and dose will be escalated until the desired effect (mobilization or peripheralization of circulating cells capable of regenerating hematopoiesis in vivo, increase of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.

The dosage of growth hormone, of one of its derivatives administered depends upon the age, sex, health and weight of the donor, type of previous or concurrent treatment, if any, frequency of the treatment and the nature of the effect desired.

Growth hormone or one of its derivatives may advantageously be administered in an amount comprised between 10 and 500 µg per kilogram of body weight, more particularly about 50 200 µg per kilogram of body weight.

A preferred dosage of Growth hormone or one of its derivatives to be administered is around 100 µg per kilogram of body weight.

Growth hormone or its derivatives may be administered alone or in conjunction or association with other factors.

Growth hormone or its derivatives may advantageously be present in a composition which comprises further one or several compound(s) chosen among the compounds belonging to the following groups: hematopoietic growth factors, cytokines, chemokines, monoclonal antibodies.

Growth hormone or its derivatives and one or several compound(s) chosen among the compounds belonging to the following groups: hematopoietic growth factors, cytokines, chemokines, monoclonal antibodies can be administered simultaneously or at different times and/or at the same site or at different site(s) and/or in the same or in a different composition or medicament

The growth factor group may comprise thrombopoietin (TPO). The cytokine group can comprise IL-1,IL-3, G-CSF, GM-CSF or SCF. Thechemokine group can comprise MIP-1α, MPIF-1. MPIF-2 or EU-2. The monoclonal antibody group can comprise anti-VLA-4 antibodies.

Preferably, Growth hormone or its derivatives is present in a composition which comprises granulocyte colony stimulating factor (G-CSF).

Preferably, Growth hormone or its derivatives is associated with G-CSF.

Growth hormone or its derivatives and G-CSF can be administered simultaneously or at different times and/or at the same site or at different site(s) and/or in the same or in a different composition or medicament.

Growth hormone or its derivatives and G-CSF may advantageously be administered separately.

G-CSF may advantageously be administered in an amount comprised between 1 and 15 µg per kilogram of body weight, more particularly between 4 and 12 µg per kilogram of body weight.

A preferred dosage of G-CSF to be administered is around 5 µg or around 10 µg per kilogram of body weight.

In a preferred embodiment, Growth hormone or one of its derivatives is administered in an amount of about 100 µg per kilogram of body weight, and G-CSF is administered in an amount of around 5 and 10 µg per kilogram of body weight.

According to the invention, the expression « administration in an amount sufficient to increase the number of circulating cells capable of regenerating hematopoiesis in vivo or to reduce the volume of blood required to be processed in order to obtain the specified target number of circulating cells capable of regenerating hematopoiesis in vivo » can mean one or several administration(s), one or several times a day and during one or several days for a cumulated amount sufficient to increase the number of circulating cells capable of regenerating hematopoiesis in vivo or to reduce the volume of blood required to be processed in order to obtain the specified target number of circulating cells capable of regenerating hematopoiesis in vivo.

The pharmaceutical compositions or compositions which are used in the uses of the invention are in a pharmaceutical acceptable form optionally combined with an acceptable carrier.

These compositions can be administered by any means that achieve their intended purposes.

The compositions used in the uses of the invention may be administered alone or in conjunction with other therapeutics directed to a disease or directed to other symptoms thereof.

The compositions used in the uses of the invention may be administered by the intravenous or the subcutaneous route, or, preferably, by the oral route.

After intravenous administration, the elimination of hGH is described by first-order kinetics with a serum half-life of 12 - 30 minutes in both animals and humans(Moore et al., 1988; Hendricks et al., 1985). Traditionally, intramuscular injection has been the method of choice as the preferred route of delivery. In humans, absorption of exogenoushGH appears to be more rapid from the intramuscular site, with a time to maximum concentration of two to three hours, compared to four to six hours after subcutaneous administration. The disappearance phase from serum has been reported to range from 12-20 hours for intramuscular administration, and 20-24 hours after subcutaneous administration (Albertsson-Wikland et al., 1986; Jorgensen et al.. 1987).

The compositions used in then uses of the invention may be administered by parenteral routes such as subcutaneous, Intravenous, intramuscular, intraperitoneal, or transdermal route or by mucosal routes such as buccal or oral route.

The composition comprising growth hormone or one of its derivatives may be administered by parenteral routes such as subcutaneous, intravenous, intramuscular, intraperitoneal, or transdermal route or by mucosal routes such as buccal or oral route.

Preferably, growth hormone or one of its derivatives either alone or in combination with G-CSF, is administered subcutaneously.

The total dose or amount required for each treatment, method or use of the invention may be administered in multiple or single dose.

The composition comprising growth hormone or one of its derivatives may be administered daily or three times a day.

Preferably, the composition comprising growth hormone or one of its derivatives is administered three times a day.

Preferably, growth hormone or one of its derivatives is administered daily or three times a day.

In a preferred embodiment, growth hormone or one of its derivatives is administered three times a day.

If a method or use of the invention comprises the administration of growth hormone or one of its derivatives and G-CSF, G-CSF is preferably administered once a day and/or intravenously.

If a use of the invention comprises the administration of growth hormone or one of its derivatives or any factor inducing growth hormone release and G-CSF, growth hormone or one of its derivatives or any factor inducing growth hormone release is preferably administered three times a day and G-CSF is preferably administered daily.

The composition comprising growth hormone or one of its derivatives may be a daily administration that can start around 20 days pre-leukapheresis.

The composition comprising growth hormone or one of its derivatives may be administered over a period of around 5 days or over a period of around 10 days or over a period of around 15 days, until leukapheresis or until the desired effect (mobilization or peripheralization of circulating cells capable of regenerating hematopoiesis in vivo, increase of the number of circulating cells capable of regenerating hematopoiesis in vivo, reduction of the number of leukapheresis required to collect sufficient amount of circulating cells for transplantation, reduction of the volume of blood required to be processed in order to obtain the specified target number of circulating cells capable of regenerating hematopoiesis in vivo) is reached.

Preferably, the composition comprising growth hormone or one of its derivatives is administered until leukapheresis and/or the desired effect (mobilization or peripheralization of circulating cells capable of regenerating hematopoiesis in vivo, increase of the number of circulating cells capable of regenerating hematopoiesis in vivo, reduction of the number of leukaphereses required to collect sufficient amount of circulating cells for transplantation, reduction of the volume of blood required to be processed in order to obtain the specified target number of circulating cells capable of regenerating hematopoiesis in vivo) is reached.

Uses of the invention are advantageously carried out before or after chemotherapy, radiotherapy, myelosuppressive therapy, transplantation or engraftment of cells capable of regenerating hematopoiesis in vivo or transplantation of bone-marrow.

Uses of the invention are advantageously carried out around 7 days after the beginning of a chemotherapeutic treatment or around 2 days after the end of a chemotherapeutic treatment.

In a preferred embodiment, growth hormone or one of its derivatives and G-CSF are administered until leukapheresis, until peripheralization or mobilization of circulating cells capable of regenerating hematopoiesis in vivo, until increase of the number of circulating cells capable of regenerating hematopoiesis in vivo, until reduction of the number of leukapheresis required to collect sufficient amount of circulating cells for transplantation, and/or until reduction of the volume of blood required to be processed in order to obtain the specified target number of circulating cells capable of regenerating hematopoiesis in vivo. In this preferred embodiment, growth hormone or one of its derivatives is preferably administered three times a day and G-CSF is preferably administered once a day.

Uses of the invention may be combined with a prior treatment called « chemopriming ». The « chemopriming » regimens which may be used are :
- high-dose cyclophosphamide (4 to 7 g/m²) for patients with breast cancer or multiple myeloma,
- ifosfamide, vinorelbine or etoposide and the like, for patients with non-Hodgkin's lymphoma or Hodgkin's disease,
- cyclophosphamide, etoposide, cisplatin (CVP) for patients with solid tumors (e.g., breast cancer).

To enhance the induction of cells capable of regenerating hematopoiesis in vivo rebound, uses of the invention are started shortly after completion of chemopriming or chemotherapy treatment and continued until completion of apheresis.

It is also noteworthy that in patients, whose bone marrow stem cell pool is significantly diminished by prior chemotherapy, an additional chemopriming regimen might impair rather than induce cells capable of regenerating hematopoiesis in vivo peripheralization. Stem cells toxic chemotherapeutic agents such as busulfan, doxorubucin, melphalan, thiotepa and possibly fludarabine (and others) should not be part of a chemopriming regimen. On the other hand, cyclophosphamide is considered the ideal chemopriming drug with the least cells capable of regenerating hematopoiesis in vivo toxicity, although cardiotoxicity (dose. > 4g/m²) and hemorrhagic cystitis are the well-known dose-limiting extramedullary side effects (To et al., 1990).

The population of blood cells enriched with cells capable of regenerating hematopoiesis in vivo obtained from the peripheral blood by the uses of the invention can be re-infused, grafted or transplanted into the same individual which is in this case the donor (autologous transplantation). Also disclosed is the transplantation into different individuals (nonautologous/heterologous transplantation).

The population of blood cells enriched with CD34 negative cells capable of regenerating hematopoiesis in vivo obtained from the peripheral blood by the uses of the invention are advantageously infused into an individual who has previously received one or several chemotherapy, radiotherapy, myelosuppressive, myeloablative or myelotoxic therapy.

Said operation of re-infusion, engraftment or transplantation belongs to the so-called Hematopoietic Stem Cells Transplantation (HSCT) procedures. HSCT is a clinical procedure in which cells capable of regenerating hematopoiesis in vivo, obtained from bone marrow or peripheral blood, are transplanted to a patient.

An autologous transplantation is a transplantation in which donor and recipient are the same individual whereas a nonautologous transplantation is a transplantation in which donor and recipient are different individuals. The method encompasses both autologous and non-autologous transplantation.

In another part, the invention provides a use of growth hormone or one of its derivatives to enhance the mobilization or peripheralization effect of G-CSF.

The invention provides a use of growth hormone or one of its derivatives to enhance mobilization of circulating CD34 negative cells capable of regenerating hematopoiesis in vivo by G-CSF, to enhance increase the number of circulating CD 34 negative cells capable of regenerating hematopoiesis in vivo by G-CSF.

Thus, the administration of growth hormone or one of its derivatives and G-CSF enhances or increases synergistically the mobilization of circulating cells capable of regenerating hematopoiesis in vivo, enhances or increases synergistically the number of circulating cells capable of regenerating hematopoiesis in vivo, reduces the number of leukaphereses required to collect sufficient amount of circulating cells for transplantation, and/or reduces the volume of blood required to be processed in order to obtain the specified target number of circulating cells capable of regenerating hematopoiesis in vivo with respect to the effect(s) obtained by administering G-CSF alone or without growth hormone or one of its derivatives.

The administration of growth hormone or one of its derivatives and G-CSF allows to use lower dose(s) of G-CSF than if G-CSF is used alone or without growth hormone or one of its derivatives or any factor inducing growth hormone release.

The administration of growth hormone or one of its derivatives and G-CSF can be carried out simultaneously or at different times and/or at the same site or at different site(s) and/or in the same or in a different composition or medicament.

Uses of the invention can be applied in many clinically important fields, namely autologous or heterologous transplantation, allogeneic or semi-allogenenic bone marrow transplantation, gene therapy, hematopoietic stem cells transplantation, transplantation of cells capable of regenerating hematopoiesis in vivo, radiotherapy, chemotherapy, myelosuppressive or myelotoxic therapy.

Uses of the invention can be applied to treat a patient who has received radiotherapy or chemotherapy, who has been transplanted with bone-marrow or cells capable of regenerating hematopoiesis in vivo, or who has received myelotoxic or myeloablative therapy.

Having now fully described the invention, it will be more readily understood through reference to the following examples that are provided by way of illustration and are not intended to be limiting of the present invention.

### FIGURE LEGENDS

- Fig. 1: shows the treatment plan of patients for cycles 1 and 3 and cycle 2, respectively. In cycles 1 and 3, patients first receive chemotherapeutic treatment with ifosphamide (IFO) and vinorelbine (VNR) and later G-CSF as mobilizing agent, whereas in cycle 2, patients first receive ifosphamide (IFO) and vinorelbine (VNR) and later a combination of G-CSF and growth hormone (GH) as mobilizing agents.
- Fig. 2: shows the development of the number of white blood cells (white blood cell counts, WBC) per µl of blood in five patients (cases 1 to 5) having received a treatment according to the treatment plan shown in Fig. 1.
- Fig. 3: depicts the development of the number of CD 34⁺ cells/µl of blood obtained in five patients (cases 1 to 5) having received a treatment according to the treatment plan shown in Fig. 1.
- Fig. 4: shows the development of the number of colony-forming cells (CFC) per milliliter of blood obtained in five patients (cases 1 to 5) having received a treatment according to the treatment plan shown in Fig. 1.
- Fig. 5: shows the development of the number of long-term culture-initiating cells (LTC-IC) per milliliter of blood obtained in four patients (cases 1 to 4) having received a treatment according to the treatment plan shown in Fig. 1.
- Fig. 6: shows a histogram depicting the peak ratios obtained in the experiments shown in Fig. 3 to 5.
- Fig. 7: shows the total number of CD 34+ cells obtained in cycles 1 to 3 in the five different patients.
- Fig. 8: shows the total number of colony forming cells (CFC) obtained in cycles 1 to 3 in the five different patients.
- Fig. 9: shows the total number of LTC-IC obtained in cycles 1 to 3 in four of the five patients.

### EXAMPLE

**Pilot clinical study showing the effect of rhGH in combination with rhG-CSF for mobilization of hematopoietic progenitor cells following chemotherapy in Hodgkin's disease patients**

### Abbreviations:

| | |
|---|---|
| - BFU-E | : burst forming unit, erythroid |
| - CFU-C | : colony forming unit, culture |
| - CFU-GM | : colony forming unit, granulocyte and macrophage |
| - CFU-Meg | : colony forming unit, megakaryocyte |
| - G-CSF | : granulocyte colony stimulating factor |
| - IGF-I | : insulin growth factor I |
| - LTC-IC | : long term culture initiating cell |
| - hGH | : human growth hormone |
| - rhG-CSF | : recombinant human granulocyte colony stimulating factor |
| - rhGH | : recombinant human growth hormone |

### Introduction

Most patients with Hodgkin disease (HD) are cured with radiation therapy, combination chemotherapy or both. However, patients who relapse after attaining complete remission and those who have primary refractory disease have poor outcomes with conventional dose salvage chemo-radiotherapy regimens. Several clinical trials using high-dose chemoradiotherapy with autologous stem cell transplantation (ASCT) show that 30% to 50% of patients appear to have been cured using this approach. Additionally, two randomized studies comparing standard-dose, second-line chemotherapy with high-dose therapy (HDT) and ASCT have demonstrated a statistically significant improvement in event-free and progression-free survival for patients treated on the HDT plus ASCT arms.

An essential prerequisite for the feasibility of HDT programs is the availability of adequate amounts of hematopoietic stem/progenitor cells to be used followingmyeloablative therapy. Optimal mobilization and collection of peripheral blood progenitor cells (PBPC) in cancer patients requires both chemotherapy and growth factor infusion. However, even under optimal conditions, some patients fail to mobilize adequate amounts of progenitor cells. Due to prior extensive chemo-radiotherapy or to disease-intrinsic factors, 30% to 50% of HD patients are hard-to-mobilize patients and cannot undergo potentially curative myeloablative therapy.

To test the hypothesis that rhGH might be useful in enhancing rhG-CSF-induced mobilization of hematopoietic progenitors in the peripheral blood, a pilot clinical study aimed at investigating the combined effects of rhGH and rhG-CSF in increasing PBPC mobilization was recently conducted in patients with relapsed/refractory HD. The objectives of the study were: (i) to assess the activity of rhGH in increasing rhG-CSF-induced mobilization of CD34+ cells, and CD34+ cell harvesting; (ii) to assess the activity of rhGH in increasing rhG-CSF-induced mobilization of immature progenitors (LTC-IC) and LTC-IC harvesting; (iii) to assess the safety and tolerability of rhGH given in combination with rhG-CSF to cancer patients following chemotherapy.

### 1. Selection criteria for the mobilization and recovery clinical studies in patients with Hodgkin's disease

### Patients

Five transplantation-eligible patients with relapsed or refractory HD treated at our Institution on a high-dose salvage chemotherapy program were included in this mobilization study.
A) Inclusion criteria :
   - Written informed consent
   - Age between 18 years and 60 years
   - Histologically confirmed Hodgkins's disease (lymphoma) undergoing high-dose salvage chemotherapy according to current INT guidelines.
B) Exclusion criteria :
   - Renal (creatinine > 1,5 N), or hepatic insufficiency (SGOT and/or SGPT > 2,5 N ; bilirubin > 1,5 N), or severe CNS or psychiatric disease.
   - Clinically significant cardiac disease or myocardiac. Left ventricular ejection fraction < 50% at rest by echocardiography assessment or < 55% by isotopic measurement.
   - Hepatitis B or C, or HIV test positive.

The main clinical characteristics of the 5 patients chosen are reported in Table 1.

**Table 1 : Clinical characteristics of the patients at the time of the study**

| Patient | | 1 | 2 | 3 | 4 | 5 |
|---|---|---|---|---|---|---|
| Age/Sex | | 28/F | 40/F | 45/M | 50/M | 50/M |
| No. of prior chemo regimens | | 1 | 1 | 2 | 1 | 1 |
| Radiotherapy | | + | + | + | + | + |
| BM involvement at relapse | | - | - | - | - | + |
| Disease extent at relapse | | | | | | |
| | Nodal | + | + | | + | |
| | Extranodal | | | | | |
| | Both | | | + | | + |

### 2. Treatment plan

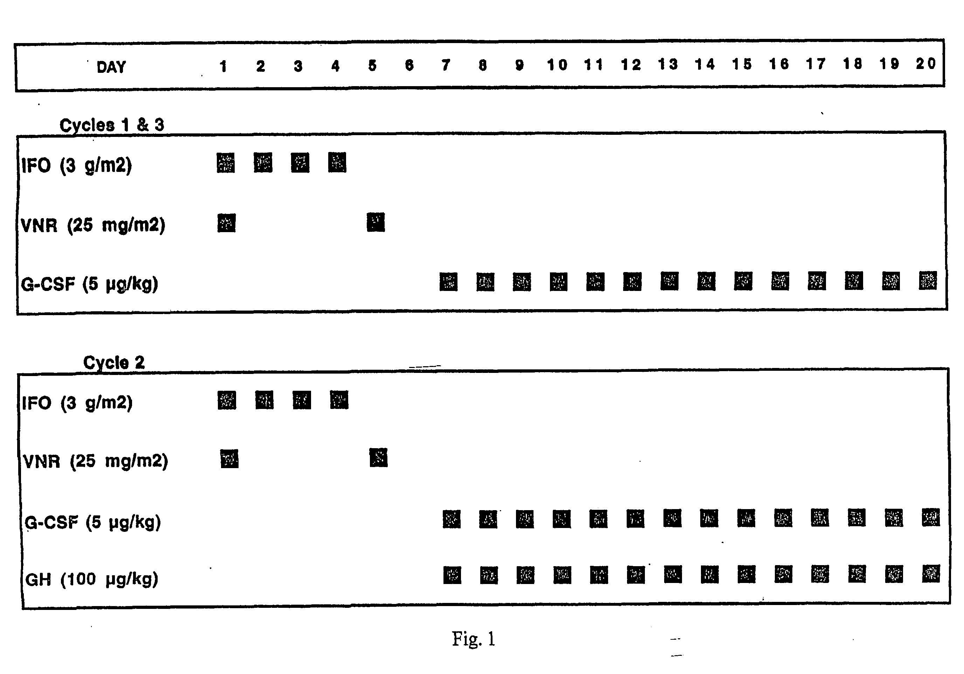

The treatment plan is illustrated in Fig. 1.

Patients received three consecutive cycles of chemotherapy including ifosphamide and vinorelbine. G-CSF was infused alone in cycle 1 and 3, and in combination with hGH in cycle 2. Thus, the kinetics of progenitor cell mobilization elicited by chemotherapy and G-CSF (cycle 1) serves as intra-patient control to assess the mobilization resulting from chemotherapy, hGH and G-CSF (cycle 2). As a consequence of cumulative bone marrow toxicity by chemotherapy, mobilization following cycle 2 was expected to be inferior to mobilization following cycle 1.

### Mobilization study with rhG-CSF (Cycle 1 and 3):

- Administration of ifosphamide (3 g/m² intravenously (iv), daily, day 1-4), uromitexan (a protecting agent against hemorrhagic cystitis) (1 g/m² iv, three times a day, day 1-4), and vinorelbine (25 mg/m² iv, daily, day 1 and 5)
- rhG-CSF administration (5 µg/kg daily, sc (subcutaneous) from day 6 until completion of CD34+ cell harvest (target cell dose is ≥8 x 10e6 CD34+ cells/kg body weight)

### Mobilization with rhGH and rhG-CSF (Cycle 2)

- Administration of ifosphamide (3 g/m2 iv, daily, day 1-4), uromitexan (1 g/m² iv, three times a day, day 1-4), and vinorelbine (25 mg/m² iv, daily, day 1 and 5)
- Co-administration of rhGH (100 µg/kg daily, sc) and rhG-CSF (5 µg/kg daily, sc) from day 6 until completion of CD34+ cell harvest (target cell dose is ≥8 x 10e6 CD34+ cells/kg body weight)

### 3. Main parameters of activity :

A) Mobilization:
   Starting from day +10, the following parameters are assessed:
   - Absolute CD34+ cell counts/µL (daily in the peripheral blood, and once in the leukapheresed cells)
   - Absolute CFU-GM counts/µl (daily in the peripheral blood, and once in the leukapheresed cells)
   - Absolute counts/µl of CFU-Meg, BFU-E, LTC-IC (in the leukapheresed cells)
B) Hematologic recovery:
   Starting from day +0, and until full and stable recovery, the following parameters are assessed:
   - Absolute granulocyte counts/µl (daily)
   - Absolute platelet counts/µl (daily)
   - Absolute erythrocyte counts/µl (daily)
   - Granulocyte nadir
   - Platelet nadir
   - Extent and duration of neutropenia
   - Extent and duration of thrombocytopenia
   - Extent and duration of hematopoietic support (platelet tranfusions, RBC transfusions)
   - Duration of infectious prophylaxis, and infections
   - Hemorrhages

### 4. Main parameters of toxicity

- Tumor growth
- Clinical and instrumental symptoms
- Laboratory tests for cardiac, liver and renal function

### 5. Study procedure

A) At baseline:
   - Complete medical history, physical examination, EKG with cardiac examination, left ventricular ejection fraction (LVEF) by multigated scintigraphic scan or echography, chest X-ray
   - Pregnancy test (if applicable)
   - HBV, HCV and HIV test
   - Complete blood count with differential
   - Absolute counts of circulating CD34+ cells and CFU-GM
   - Blood chemistry (transaminases, serum alkaline phosphatase, gammaGT, LDH, total bilirubin, BUN, creatinine, glycemia, Na, K, Ca, P, uric acid, total protein, albumin, cholesterol, triglycerides
   - Bilateral bone marrow biopsy
   - Informed consent
B) Mobilization assessment:
   - Daily assessment of CD34+ cells/µL and CFU-GM/µL in the peripheral blood, from day +10 until leukapheresis
   - Total yield of CD34+ cells, CFU-GM, BFU-E, CFU-Meg, as well as LTC-IC in leukapheresed cells
   - Toxicity assessment through clinical and instrumental examinations (EKG, chest X-ray, and other examinations as required)
   - Measurement and evaluation of all tumor parameters after ending the mobilization study
C) Hematologic recovery assessment:
   - Daily assessment of WBC, RBC and platelet counts
   - Number of platelet transfusions
   - Number of RBC transfusions
   - Type and severity of fever and documented infections
   - Clinical and instrumental assessment of toxicities other than hematological

### 6. Material and methods

### Chemotherapy and PBPC mobilization

After providing written, informed consent, patients received three consecutive cycles of ifosphamide (IFO, 3 g/m² iv, QD, day 1-4) and vinorelbine (VNR, 25 mg/m² iv, QD, day 1 and 5) (Figure 1). After the first and third cycle of IFO/VNR chemotherapy, PBPCs were mobilized using rhG-CSF alone (5 µg/kg, QD, sc), whereas after the second cycle of IFO/VNR chemotherapy, PBPC mobilization was elicited by rhG-CSF and rhGH (100 µg/kg, QD, sc). Thus, the kinetics of progenitor cell mobilization elicited by chemotherapy andrhG-CSF (cycle 1) served as intra-patient control to assess the mobilization resulting from chemotherapy, rhGH and rhG-CSF (cycle 2). Mobilization therapy was administered from day 7 until the completion of CD34+ cell harvest (target cell dose was ≥8 x 10⁸ CD34+ cells/kg body weight). PBPC collections were started when circulating CD34+ cells were ≥20/µL. Parameters to monitored on a daily basis starting when white blood cell (WBC) counts were ≥1,000/µl and until completion of leukapheresis included: WBC counts, CD34+ cells, committed hematopoietic progenitors (CFU-Mix, BFU-E, CFU-GM) and primitive hematopoietic progenitors (LTC-IC).

### Flow cytometry

CD34+ cells were detected by direct immunofluorescence. Briefly, buffy-coat cells (1 x 10⁶) incubated with either fluorescein isothiocyanate (FITC)-conjugated-HPCA-2 monoclonal antibody (Becton-Dickinson, San Jose, CA) or with a mouse IgG1-FITC antibody (B-D) as negative control were analyzed on a FACScan laser flow cytometry system (B-D) equipped with a Macintosh PowerMac G3 personal computer (Apple Computer Inc., Cupertino, CA, USA) using Cell Quest (B-D) software.

### CFU-Mix, BFU-E, CFU-GM Assay

The assay for CFU-Mix, BFU-E, and CFU-GM was carried out as previously described. Briefly, 1 to 5 x 10⁴ nucleated cells from mobilized blood were plated in 35-mm Petri dishes in 1-ml aliquots of IMDM containing: 30% fetal bovine serum (FBS, Stem Cell Technologies, Vancouver, Canada); 10⁻⁴ M 2-mercaptoethanol (Gibco, Grand Island, NY, USA); and 1.1% (w/v) methylcellulose. Cultures were stimulated with interleukin-3 (10 ng/ml, Sandoz, Basel, Switzerland), granulocyte colony-stimulating factor (10 ng/ml, Amgen Inc., Thousand Oaks, CA), granulocyte-macrophage colony-stimulating factor (10 ng/ml, Sandoz) and erythropoietin (3 U/ml, Amgen Inc.). Progenitor cell growth was evaluated according to previously published criteria after 14-18 days of incubation (37°C, 5% CO₂) in a humidified atmosphere. Four plates were scored for each data point and the results were expressed as the mean ± SEM.

### LTC-IC Assay

The long-term culture-initiating cell (LTC-IC) assay was performed as previously described. Briefly, test cells were resuspended in complete medium consisting of alpha-medium (Gibco) supplemented with FBS (12.5%), horse serum (12.5%), L-glutamine (2 mM), 2-mercaptoethanol (10⁻⁴ M), inositol (0.2 mM), folic acid (20 µM) and freshly dissolved hydrocortisone (10⁻⁶ M). Test cell (5 x 10⁶ nucleated cells) suspension was seeded into cultures containing a feeder layer of irradiated (8,000 cGy) murine M2-10B4 cells (3 x 10⁴/cm², kindly provided by Dr. C. Eaves, Terry Fox Laboratory, Vancouver, Canada) engineered by retroviral gene transfer to produce human IL-3 and G-CSF. After 5 weeks in culture, nonadherent cells and adherent cells harvested by trypsinization were pooled, washed, and assayed together for clonogenic cells in standard methylcellulose cultures at an appropriate concentration. The total number of clonogenic cells (i.e., CFU-Mix plus BFU-E plus CFU-GM) present in 5-week-old LTC provides a relative measure of the number of LTC-IC originally present in the test suspension. Absolute LTC-IC values were calculated by dividing the total number of clonogenic cells by 4, which is the average output of clonogenic cells per LTC-IC, according to limiting dilution analysis studies reported by others.

### 7. Results of the pilot clinical study

Fig. 1 illustrates the treatment plan the patients were subjected to. Five patients with relapsed Hodgkin's disease (Table + above) received three cycles of treatment (Fig. 1). The first and third cycle consisted in the administration of ifosphamide at 3 g/m², administered intravenously (iv) daily for days 1 to 4 and administration of vinorelbine 25 mg/m² iv, daily, on days 1 and 5. Subsequently, patients were treated with recombinant human granulocyte colony-stimulating factor (rhG-CSF) at 5 µg/kg sc, daily, starting on day 7. Treatment was pursued till day 20, or until completion of leukapheresis, if the peak of CD34+ cells was reached before.

Cycle 2 consisted in the same treatment plan for iphosphamide and vinorelbine, which was then followed by administration of recombinant human granulocyte colony-stimulating factor (rhG-CSF, 5 µg/kg iv) plus recombinant human growth hormone (rhGH, 100 µg/kg sc), both administered daily, starting on day 7 till 20 or until completion of leukapheresis.

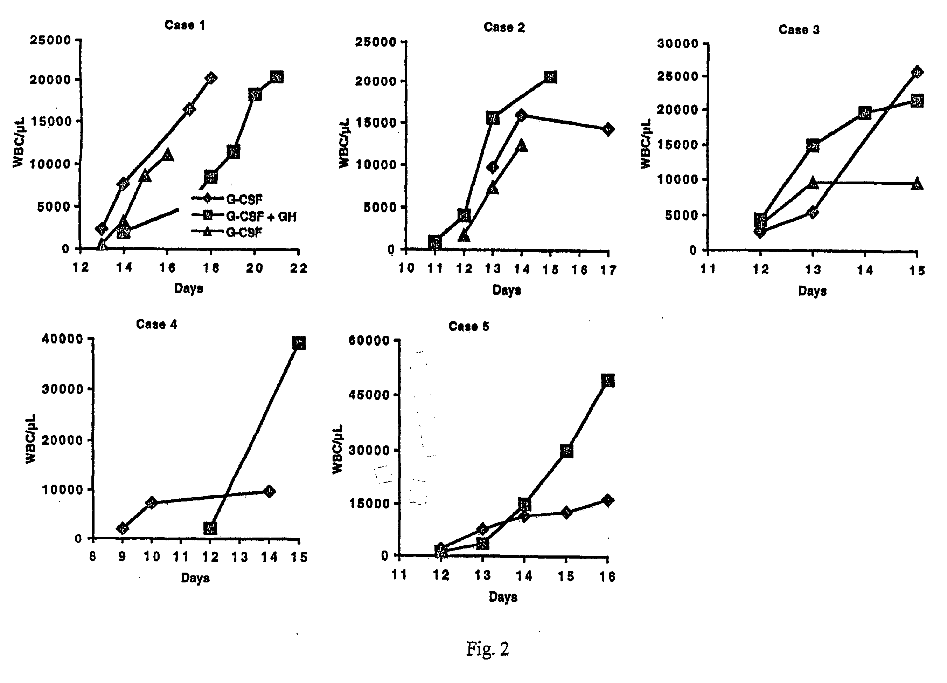

The results for the most relevant parameters measured in the first five patients participating in the clinical study are shown in Fig. 2 to 5. In all patients, cycle 1 (using G-CSF alone as mobilizing agent) represented an intra-patient control. It served for assessing the ability of hGH + G-CSF (cycle 2) to enhance progenitor/stem cell mobilization induced by chemotherapy and G-CSF. Cycle 3 then showed the mobilization capacities of the patient with G-CSF alone again.

It should be noted that all patients involved in this pilot study were included into this study because they were classified as being "bad" mobilizers, meaning that the mobilization of hematopoietic stem cells was reduced in each of the five patients with regard to average patients undergoing chemotherapy having the same illness. The threshold is a number of about 10 CD34+ cells/µl or less than about 3 x 10e6 cells/µl. It should be further noted that the second and third leukaphereses are usually hampered by the previous one(s), i.e. the number of cells, in particular the number of mobilized stem cells is expected to become lower and lower the more cycles of treatment and leukaphereses the patient is submitted to.

Fig. 2 illustrates the White blood cell (WBC) count per microliter in the patients with relapsed Hodgkin's disease receiving treatment according to the three cycle treatment plan as outlined above. White blood cell (WBC) counts recorded in the 5 patients after each chemotherapy cycle are shown in Figure 2. Addition of rhGH at the second cycle failed to substantially affect the kinetics of WBC counts.

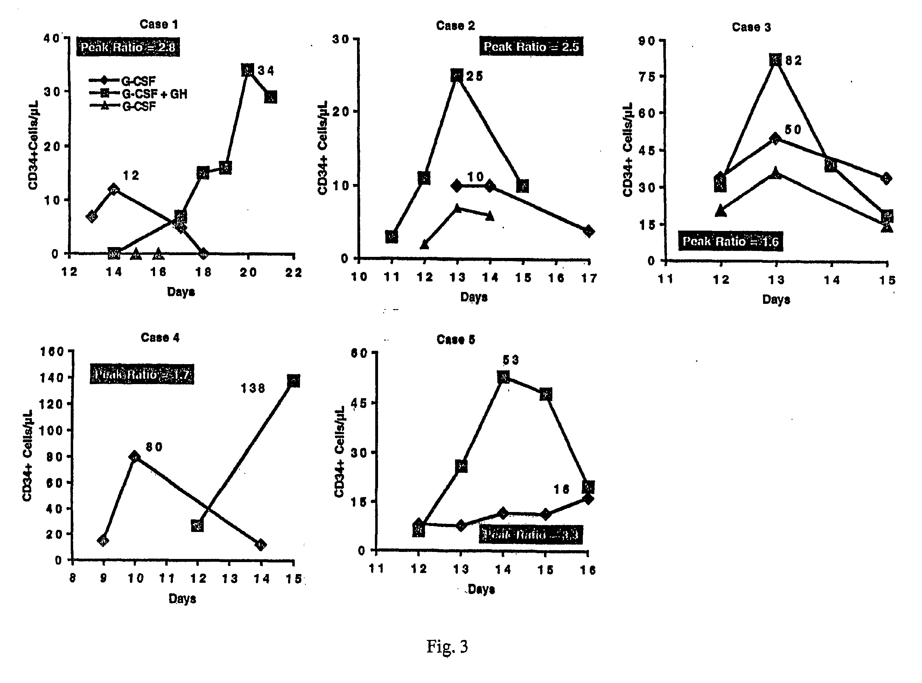

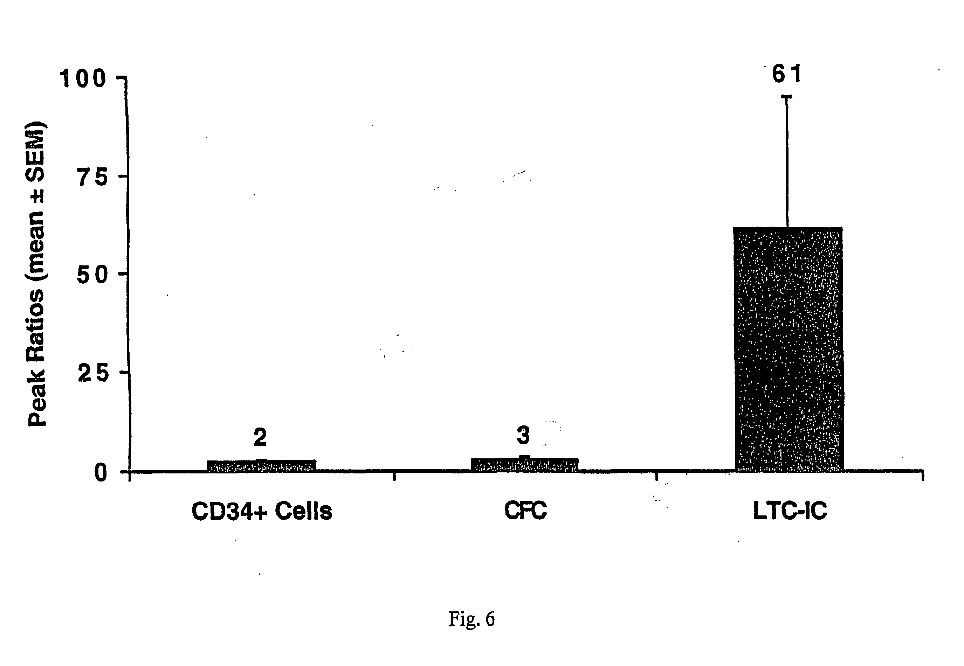

Fig. 3 shows the kinetics of mobilization of CD34⁺ cells per microliter of blood in relapsed Hodgkin's disease patients receiving the above treatment. The peak ratio represents the maximum CD34⁺ cell value per µl of blood afterrhG-CSF + rhGH divided by the maximum CD34⁺ cell value per µl blood after rhG-CSF. As shown, in 5 out of 5 cases the peak values of CD34+ cells detected following rhG-CSF plus rhGH were higher as compared to those observed after rhG-CSF alone. The mean peak ratio, i.e., maximum CD34+ cell value after rhG-CSF + rhGH/maximum CD34+ cell value after rhG-CSF, was 2.38 (range, 1.6 to 3.3). In all of the patients, the maximal amounts of CD34+ cells retrieved by leukapheresis were much higher after administration of the combination ofhGH and G-CSF (cycle 2) as compared to G-CSF alone (cycles 1 and 3). The peak rations are summarized in Table 2 below and illustrated in Fig. 6. They vary between 1,6 and 3,3 and have a mean value of 2,38, meaning that the combination treatment resulted in more than a doubling of the mobilized CD34+ cells. This result is particularly noteworthy, because the patients were classified as bad mobilizers.

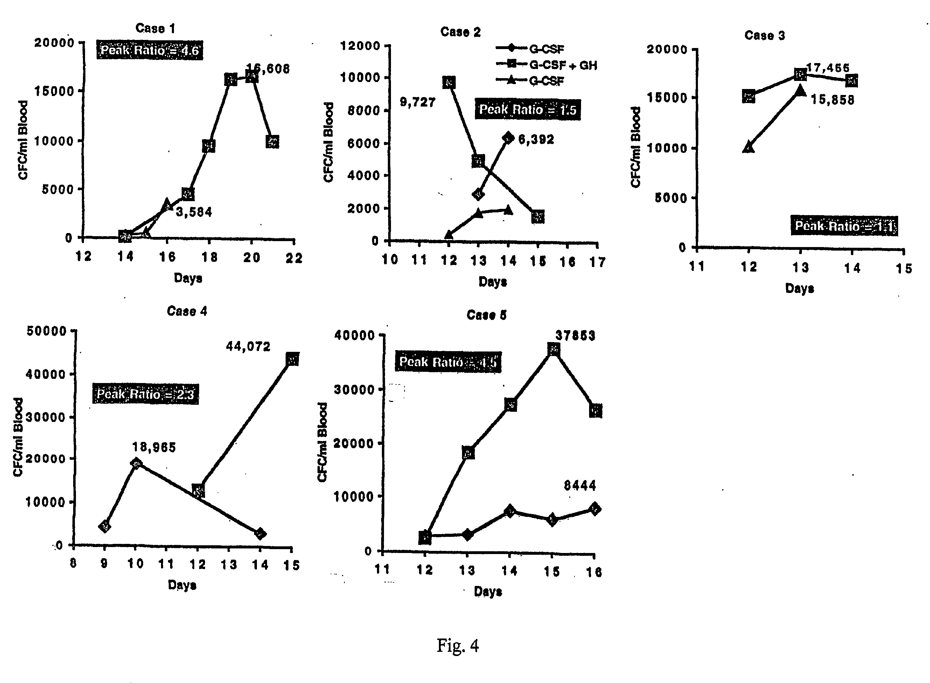

Fig. 4 shows the kinetics of mobilization of total colony-forming cells (CFC) per milliliter of blood in patients with relapsed Hodgkin's disease receiving the treatment outlined above. Total CFC include granulocyte-macrophage CFC (CFU-GM), erythroid burst-forming unit (BFU-E) and multipotent CFC (CFU-Mix). Data are expressed as mean values derived from quadruplicate cultures. The peak ratios represent maximum CFC value per ml blood after rhG-CSF + rhGH divided by the maximum CFC value per ml blood after rhG-CSF.

In all instances, CFC mobilization was improved by addition ofrhGH to rhG-CSF with a mean peak ratio of 2.8 (range, 1.1 to 4.6).

The results of the analysis of the colony forming cells supports the positive results of the analysis of CD34+ cells. The average peak ratio calculated in this experimental series (see Table 2 below) is 2,8, meaning that the combined treatment almosttriplicated the yield of CFC.

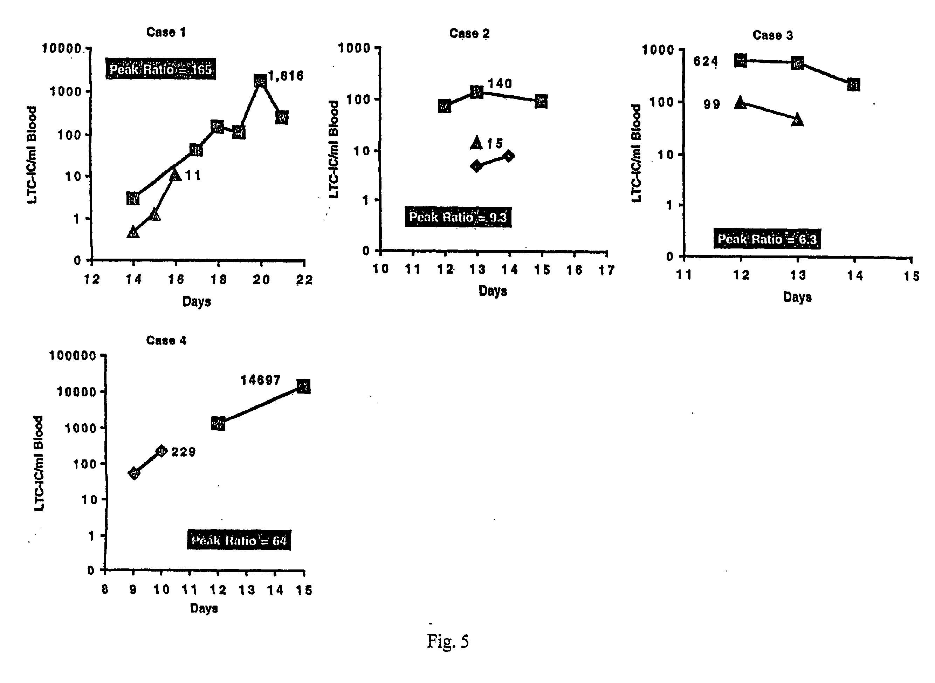

Fig. 5 shows the mobilization of long-term culture-initiating cells (LTC-IC) per milliliter of blood in patients with relapsed Hodgkin's disease receiving the treatment outlined above. Again, data are expressed as mean values derived from quadruplicate cultures. The peak ratio represents the maximum LTC-IC value per ml blood afterrhG-CSF + rhGH divided by the maximum LTC-IC value per ml blood after rhG-CSF. Interestingly, addition of rhGH to rhG-CSF significantly improved PBPC mobilization with a mean peak ratio of 49 (range, 2 to 165).

The amount of LTC-IC is assessed in the Long-Term Culture-Initiating Cell (LTC-IC) assay. By using the technique of long-term culture (LTC) a sustained production of lymphomyeloid cells can be readily achieved in vitro, provided that a stromal layer is present, by placing hematopoietic cells in liquid culture at relatively high cell concentration, with appropriate supplements, temperature and feeding conditions. The LTC system, based on the re-establishment in vitro of the essential cell types and mechanism responsible for the localized and sustained production of hematopoietic cells in the marrow in vivo, offers an approach able to investigate not only the proliferative and differentiation events, but also self-renewal of any clonogenic cell types. A 5- to 8-week time period between initiating LTC and assessing clonogenic progenitor numbers allows to quantitate a primitive hematopoietic cell, the so-called "long-term culture-initiating cell" (LTC-IC) which has self-renewal potential and phenotype characteristics overlapping with those of transplantable murine in vivo repopulating cells (Sutherland et al., 1989; Petzer et al., 1996 and Lemieux et al., 1995)). Limiting dilution assays allow to calculate the frequency of LTC-IC and their proliferative potential (number of CFU-C generated by each LTC-IC).

To perform the LTC-IC assay, peripheral blood nucleated cells were resuspended in complete medium consisting of alpha-medium (Gibco) supplemented with fetal bovine serum (12.5%), horse serum (12.5%), L-glutamine (2 mM), 2-mercaptoethanol (10⁻⁴ M), inositol (0.2 mM), folic acid (20 µM) and freshly dissolved hydrocortisone (10⁻⁶ M). Blood cells (5 x 10⁶) are then seeded into cultures containing a feeder layer of irradiated (8,000 cGy) murine M2-10B4 cells (3 x 10⁴/cm²) engineered by retroviral gene transfer to produce human IL-3 and G-CSF (Sutherland et al., 1994). After 5 weeks in culture, nonadherent cells and adherent cells harvested by trypsinization were pooled, washed, and assayed together for clonogenic cells in standard methylcellulose cultures at an appropriate concentration. The total number of clonogenic cells (i.e., CFU-Mix plus BFU-E plus CFU-GM) present in 5-week-old LTC provides a relative measure of the number of LTC-IC originally present in the test suspension (Udomsakdi et al, 1992). Absolute LTC-IC values were calculated by dividing the total number of clonogenic cells by 4, which is the average output of clonogenic cells per LTC-IC, according to limiting dilution analysis studies (Udomsakdi et al., 1992).

At least a portion of LTC-IC does not present the CD34 antigen on their cell surface, and thus lack the classical marker of primitive hematopoietic progenitor cells. There are also indications that expression of the CD34 antigen may switch from positive to negative or vice versa, be it in culture or in vivo.

The results obtained in the LTC-IC assay support very efficient activity of a combination of hGH and G-CSF as mobilizing agents of primitive hematopoietic stem cells. The amount of mobilized LTC-IC was much higher than expected. As can be taken from Fig. 5, the amount of LTC-IC per milliliter of blood retrieved from the patient byleukapheresis was clearly superior after treatment of the patients with a combination ofrhG-CSF and rhGH (cycle 2) as compared to the treatment with rhG-CSF alone (cycles 1 and 3). The results for patient 5 were not available.

The peak values (see Table 2, Fig. 6) show a great variety between the individual patients. They vary between 165 and 6,5, the mean value being 61. The pronounced effect by hGH and G-CSF on LTC-IC mobilization is clearly shown.

The superior activity of the combination treatment with GH and G-CSF on the mobilization of LTC-IC is especially noteworthy and was totally unexpected, first because all patients participating in the study were qualified as bad mobilizers, and second because the unexpected high amounts of primitive hematopoietic precursor cells were observed in the second cycle of leukapheresis of the patient, which is usually already hampered by the first cycle of mobilization.

**Table 2: Peak ratios for CD34+ cells, CFC and LTC-IC**

| Cell type | Case 1 | Case 2 | Case 3 | Case 4 | Case 5 | Average |
|---|---|---|---|---|---|---|
| CD 34 + | 2,8 | 2,5 | 1,6 | 1,7 | 3,3 | 2,38 |
| CFC | 4,6 | 1,5 | 1,1 | 2,3 | 4,5 | 2,8 |
| LTC-IC | 165 | 9,3 | 6,3 | 64 | n.d.* | 61,15 |

| | | | | | | |
|---|---|---|---|---|---|---|
| * not determined | | | | | | |

Further, the results obtained using the LTC-IC assay are remarking with regard to the results obtained measuring the CD34+ cell count (Fig. 3). Whereas the combined administration of GH and G-CSF caused an enhancement of mobilization of CD34+ cells by two- to three-fold, which is already a major improvement, the impact of the combined treatment on the mobilization of LTC-IC is in the range of around 50fold.

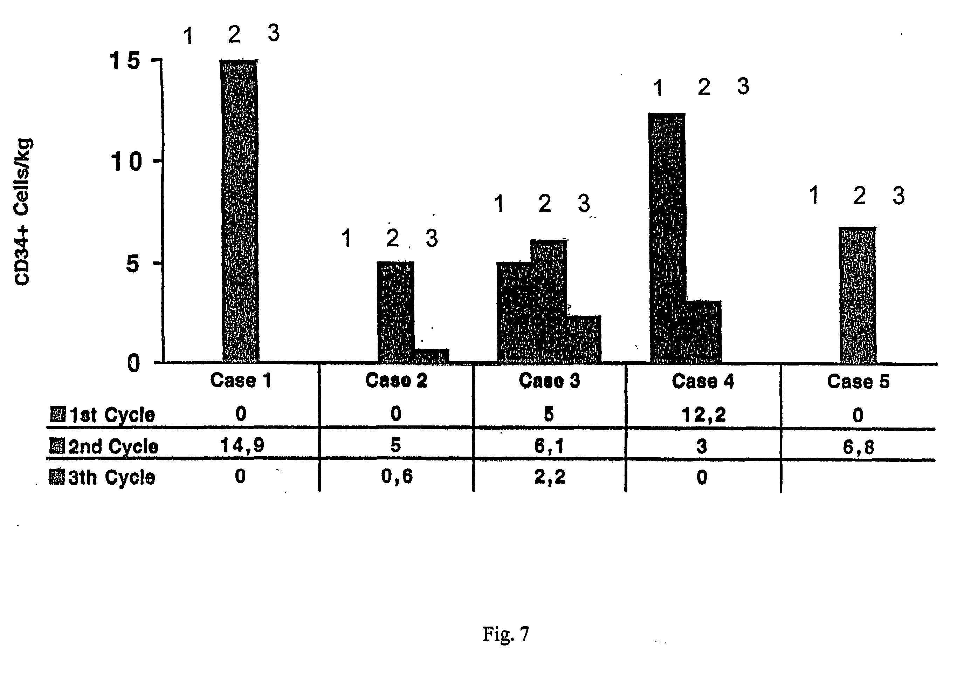

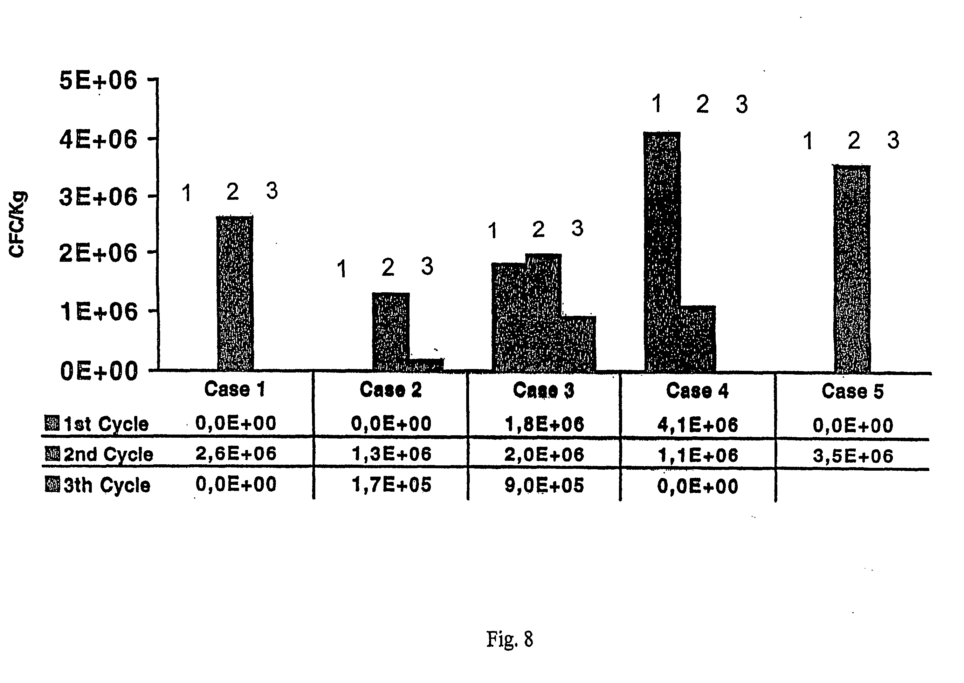

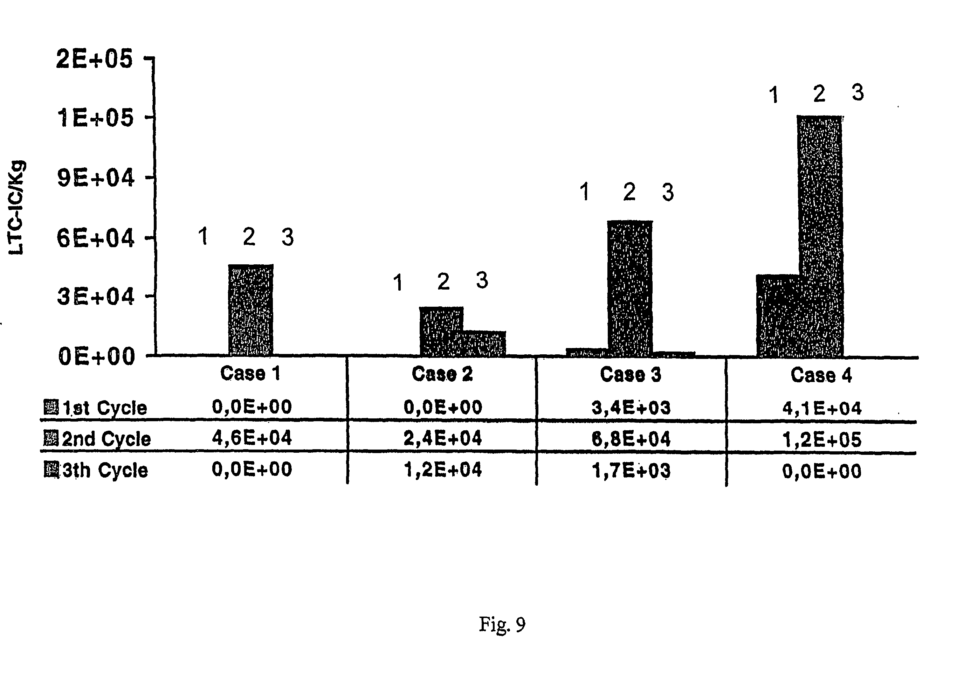

Fig. 7 to 9 show the total amounts of CD34+ cells per kilogram of body weight of the patient (Fig. 7), CFC per kilogram (Fig. 8) and LTC-IC per kilogram (Fig. 9) retrieved during cycles 1, 2 and 3. Confirming the above-outlined results from the kinetics studies, in every case cycle 2 was the cycle in which the highest amount of CD34+ cells, CFC or LTC-IC were mobilized and collected during leukapheresis.

### PBPC collection

After the first chemotherapy cycle, only 2 out of 5 patients achieved CD34+ cell levels allowing PBPC collection whereas no collection could be performed in the remaining 3 patients. In striking contrast, 5 out of 5 patients were eligible for PBPC collection upon mobilization with rhG-CSF and rhGH. However, due to the amount of CD34+ cells collected after the first cycle (12 x 10⁶/kg body weight), patient #4 underwentleukapheresis at the first cycle only. Collection data are summarized in Table 2.

### Side effects

During rhGH injection, one patient with reduced glucose tolerance showed a transient hyperglycemia requiring insulin therapy. Hyperglycemia was well controlled by insulin and did not require rhGH discontinuation so that the patient could successfully complete PBPC mobilization and collection. Blood glucose levels decreased to normal values two days after stopping rhGH therapy. No other specific side effect could be ascribed to rhGH therapy.

**Table 2 : CD34+ cells/kg body weight collected after each cycle of IFO/VNR chemotherapy**

| Cycle | Cytokine(s) | Case 1 | Case 2 | Case 3 | Case 4 | Case 5 |
|---|---|---|---|---|---|---|
| 1 | rhG-CSF | 0 | 0 | 5 x 10⁶ | 12 x 10⁶ | 0 |
| 2 | rhG-CSF + rhGH | 15 x 10⁶ | 5 x 10⁶ | 6 x 10⁶ | nd | 7 x 10⁶ |
| 3 | rhG-CSF | 0 | 0.6 x 10⁶ | 2.2 x 10⁶ | nd | Nd |

### Conclusions

The new findings described above represent a major improvement of mobilization technique. Mobilization of progenitor cells plays a pivotal role in at least three clinically important fields, namely autologous bone marrow transplantation, allogeneic and semi-allogeneic bone marrow transplantation, and gene therapy.

In normal donors, mobilization is accomplished by a treatment with G-CSF for a few days. So far substitutes or adjuncts to G-CSF either failed to improve the mobilization achieved with G-CSF alone, or resulted in a limited improvement outweighed by a substantially increased toxicity (see combinations of rhG-CSF with either rhSCF or rhIL3). Mobilization with G-CSF alone has both quantitative and qualitative limitations. Quantitatively, only 50% of the donors of progenitor cells can undergo one single leukapheresis. The remaining half either fails to yield an optimal amount of progenitors (i.e. ≥5 x 10e6 CD34+ cells/kg), or requires multiple leukaphereses (up to 4) over consecutive days. Any procedure applicable to normal donors, and capable of increasing the yield of circulating progenitors in the absence of added toxicity, has a profound impact on mobilization and procurement of progenitor/stem cells.

Another unexpected finding was the short-term activity of the treatment. Given the extremely short period of time hGH and G-CSF had to be administered (maximal 13 days), no serious adverse effects were observed nor expected. In most of the cases, treatment could be terminated much earlier since the peak value of CD 34+ cells was already observed before the end of the treatment. Leukapheresis was then directly carried out and further treatment stopped until cycle 3.

### REFERENCES

Adelman, J.P., Hayflick, J.S., Vasser, M., and Seeburg, P.H. (1983). In vitro deletional mutagenesis for bacterial production of the 20,000- dalton form of human pituitary growth hormone. DNA 2, 183-193.
Albertsson-Wikland, K., Westphal, O., and Westgren, U. (1986). Daily subcutaneous administration of human growth hormone in growth hormone deficient children. Acta Paediatr.Scand. 75, 89-97.
Alexander, W.S. (1998). Cytokines in hematopoiesis. Int.Rev.Immunol. 16, 651-682.
Anderlini, P. and Korbling, M. (1997). The use of mobilized peripheral blood stem cells from normal donors for allografting. Stem.Cells 15, 9-17.
Becker, G.W., Bowsher, R.R., Mackellar, W.C., Poor, M.L., Tackitt, P.M., and Riggin, R.M. (1987). Chemical, physical, and biological characterization of a dimeric form of biosynthetic human growth hormone. Biotechnol.Appl.Biochem. 9, 478-487.
Becker, G.W., Tackitt, P.M., Bromer, W.W., Lefeber, D.S., and Riggin, R.M. (1988). Isolation and characterization of a sulfoxide and a desamido derivative of biosynthetic human growth hormone. Biotechnol.Appl.Biochem. 10, 326-337.
Bewley, T.A. and Li, C.H. (1975). The chemistry of human pituitary growth hormone. Adv. Enzymol. Relat. Areas. Mol. Biol. 42:73-166, 73-166.
Cunningham, B.C., Mulkerrin, M.G., and Wells, J.A. (1991). Dimerization of human growth hormone by zinc. Science 253, 545-548.
DeNoto, F.M., Moore, D.D., and Goodman, H.M. (1981). Human growth hormone DNA sequence and mRNA structure : possible alternative splicing. Nucleic.Acids.Res. 9, 3719-3730.
Derfalvi, B., Sallai, P., Nemet, K., Szalai, C., Kenesei, E., Tulassay, T., and Falus, A. (1998). [The in vitro effect of recombinant human growth hormone on lymphocyte and granulocyte function in healthy and uremic children]. Orv.Hetil. 139, 1847-1850.
Fischer, A., Haddad, E., Jabado, N., Casanova, J.L., Blanche, S., Le Deist, F., and Cavazzana-Calvo, M. (1998). Stem cell transplantation for immunodeficiency. Springer Semin.Immunopathol. 19, 479-492.
Geffner, M. (1997). Effects of growth hormone and insulin-like growth factor I on T- and B-lymphocytes and immune function. Acta Paediatr.Suppl. 423 :76-9, 76-79.
Gertler, A., Shamay, A., Cohen, N., Ashkenazi, A., Friesen, H.G., Levanon, A., Gorecki, M., Aviv, H., Hadary, D., and Vogel, T. (1986). Inhibition of lactogenic activities of ovine prolactin and human growth hormone (hGH) by a novel form of a modified recombinant hGH. Endocrinology 118, 720-726.
Gianni, A.M., Bregni, M., Siena, S., Villa, S., Sciorelli, G.A., Ravagnani, F., Pellegris, G., and Bonadonna, G. (1989). Rapid and complete hemopoietic reconstitution following combined transplantation of autologous blood and bone marrow cells. A changing role for high dose chemo-radiotherapy ? Hematol.Oncol. 7, 139-148.
Goeddel, D.V., Heyneker, H.L., Hozumi, T., Arentzen, R., Itakura, K., Yansura, D.G., Ross, M.J., Miozzari, G., Crea, R., and Seeburg, P.H. (1979). Direct expression in Escherichia coli of a DNA sequence coding for human growth hormone. Nature 281, 544-548.
Haas R, Murea S (1995): The role of granulocyte colony-stimulating factor in mobilization and transplantation of peripheral blood progenitor and stem cells [published erratum appears in Cytokines Mol Ther 1996 May;2(1):136] Cytokines Mol Ther 1:4 249-70.
Heather J. et al. Blood 74 ; 1563-1570 (1989).
Hendricks, C.M., Eastman, R.C., Takeda, S.,Asakawa, K., and Gorden, P. (1985). Plasma clearance of intravenously administered pituitary human growth hormone: gel filtration studies of heterogeneous components. J.Clin.Endocrinol.Metab. 60, 864-867.
Henry, D. (1997). Haematological toxicities associated with dose-intensive chemotherapy, the role for and use of recombinant growth factors. Ann.Oncol. 8 Suppl 3:S7-10, S7-10.
Jorgensen, J.O., Flyvbjerg, A., Dinesen, J., Lund, H., Alberti, K.G., Orskov, H., and Christiansen, J.S. (1987). Serum profiles and short-term metabolic effect of pituitary and authentic biosynthetic human growth hormone in man. Adouble-blind cross-over study. Acta Endocrinol.(Copenh.) 116, 381-386.
Jorgensen, K.D., Monrad, J.D., Brondum, L., and Dinesen, B. (1988). Pharmacokinetics of biosynthetic and pituitary human growth hormones in rats. Pharmacol.Toxicol. 63, 129-134.
Kimata, H. and Yoshida, A. (1994). Effect of growth hormone and insulin-like growth factor-I on immunoglobulin production by and growth of human B cells. J.Clin.EndocrinoLMetab. 78, 635-641.
Kotzmann, H, Riedl, M., Clodi, M., Barnas, A., Kaider, A., Höcker, P. and Luger, A. (1996). The influence of growth hormone substitution therapy on erythroid and myeloid progenitor cell and on peripheral blood cells in adult patients with growth hormone deficiency. Eur. J. of Clin. Invest. 26, 1175-1181.
Larsson, K., Bjorkstrand, B., and Ljungman, P. (1998). Faster engraftment but no reduction in infectious complications after peripheral blood stem cell transplantation compared to autologous bone marrow transplantation. Support.Care Cancer 6, 378-383.
Lemieux ME, Rebel VI, Landsorp PM, et al. Characterization and purification of a primitive hematopoietic cell type in adult mouse marrow capable of lymphomyeloid differentiation in long-term marrow "switch" cultures. Blood 86: 1339, 1995
Lemoli RM, Tafuri A, Fortuna A, Catani L, Rondelli D, Ratta M, Tura S (1998). Biological characterization of CD34+ cells mobilized into peripheral blood. Bone Marrow Transplant 22 Suppl 5, 47-50.
Lewis, U.J., Peterson, S.M., Bonewald, L.F., Seavey, B.K., and VanderLaan, W.P. (1977). An interchain disulfide dimer of human growth hormone. J.Biol.Chem. 252, 3697-3702.
Lewis, U.J., Singh, R.N., Bonewald, L.F., Lewis, L.J., and VanderLaan, W.P. (1979). Human growth hormone: additional members of the complex. Endocrinology 104, 1256-1265.
Lewis, U.J., Singh, R.N., Bonewald, L.F., and Seavey, B.K. (1981). Altered proteolytic cleavage of human growth hormone as a result of deamidation. J.Biol.Chem. 256, 11645-11650.
Lewis, U.J., Singh, R.N., VanderLaan, W.P., and Tutwiler, G.F. (1977). Enhancement of the hyperglycemic activity of human growth hormone by enzymic modification. Endocrinology 101, 1587-1603.
Merchav, S., Tatarsky, I., and Hochberg, Z. (1988). Enhancement of erythropoiesis in vitro by human growth hormone is mediated by insulin-like growth factor I. Br.J.Haematol. 70, 267-271.
Moore, J.A., Rudman, C.G., MacLachlan, N.J., Fuller, G.B., Burnett, B., and Frane, J.W. (1988). Equivalent potency and pharmacokinetics of recombinant human growth hormones with or without an N-terminal methionine. Endocrinology 122, 2920-2926.
Petzer AL, Hogge DE, Lansdorp PM, Reid DS, Eaves CJ. Self-renewal of primitive human hematopoietic cells (long-term-culture-initiating cells) in vitro and their expansion in defined medium. Proc Natl Acad Sci (USA) 93: 1470, 1996
Siena et al. Blood 74 ; 1905-1914 (1989)
Siena et al. Blood 77 ; 400-409 (1991)
Singh, R.N., Seavey, B.K., Rice, V.P., Lindsey, T.T., and Lewis, U.J. (1974). Modified forms of human growth hormone with increased biological activities. Endocrinology 94, 883-891.
Stolar, M.W., Ambum, K., and Baumann, G. (1984). Plasma "big" and "big-big" growth hormone (GH) in man : an oligomeric series composed of structurally diverse GH monomers. J.Clin.Endocrinol.Metab. 59, 212-218.
Stolar, M.W. and Baumann, G. (1986). Big growth hormone forms in human plasma : immunochemical evidence for their pituitary origin. Metabolism 35, 75-77.
Sutherland HJ, Eaves CJ, Eaves AJ, Dragowskas W, Lansdorp PM. Characterization and partial purification of human marrow cells capable of initiating long-term hematopoiesis in vitro. Blood 74: 1563, 1989
Sutherland HJ, Eaves CJ, Lansdorp PM, Phillips GL, Hogge DE. Kinetics of committed and primitive blood progenitor mobilization after chemotherapy and growth factor treatment and their use in autotransplants. Blood 83: 3808, 1994
Sutherland HJ, Hogge DE, Cook D, Eaves CJ. Alternative mechanisms with and without steel factor support primitive human hematopoiesis. Blood 81: 1465, 1993
Thorlacius-Ussing, O. (1987). Zinc in the anterior pituitary of rat : a histochemical and analytical work. Neuroendocrinology. 45, 233-242.
Tian, Z.G., Woody, M.A., Sun, R., Welniak, L.A., Raziuddin, A., Funakoshi, S., Tsarfaty, G., Longo, D.L., and Murphy, W.J. (1998). Recombinant human growth hormone promotes hematopoietic reconstitution after syngeneic bone marrow transplantation in mice. Stem.Cells 16, 193-199.
To LB, Shepperd KM, Haylock DN et al. Single high doses of cyclophosphamide enable the collection of high numbers of hemopoietic stem cells from the peripheral blood. Exp Hematol (1990) ; 18, 442-447.
Udomsakdi C, Lansdorp PM, Hogge DE, Reid DS, Eaves AC, Eaves CJ. Characterization of primitive hematopoietic cells in normal humal peripheral blood. Blood 80: 2513, 1992
Valerio, G., Di Maio, S., Salerno, M., Argenziano, A., Badolato, R., and Tenore, A. (1997). Assessment of red blood cell indices in growth-hormone-treated children. Horm.Res. 47, 62-66.
Van Hoef, M.E. (1998). Haematological recovery after high-dose consolidation chemotherapy with peripheral blood progenitor cell rescue: the effects of the mobilization regimen and post-transplant growth factors. Neth.J.Med. 52, 30-39.
Vihervuori, E., Virtanen, M., Koistinen, H., Koistinen, R., Seppala, M., and Siimes, M.A. (1996). Hemoglobin level is linked to growth hormone-dependent proteins in short children. Blood 87, 2075-2081.
Waters, T.M., Bennett, C.L., Pajeau, T.S., Sobocinski, K.A., Klein, J.P., Rowlings, P.A., and Horowitz, M.M. (1998). Economic analyses of bone marrow and blood stem cell transplantation for leukemias and lymphoma : what do we know ? Bone Marrow Transplant. 21, 641-650.
Weaver, A. and Testa, N.G. (1998). Stem cell factor leads to reduced blood processing during apheresis or the use of whole blood aliquots to support dose-intensive chemotherapy. Bone Marrow Transplant. 22, 33-38.

## Claims

1. Use of growth hormone or one of its derivatives for the manufacture of a medicament for increasing, in a patient in need of hematopoietic regeneration, the number of CD34 negative pluripotent peripheral blood cells capable of regenerating hematopoiesis, so as to provide said CD34 negative pluripotent peripheral blood cells in an amount sufficient for autologous transplantation.

2. Use according to claim 1, wherein the medicament further comprises one or several compound(s) chosen among the following groups of compounds: hematopoietic growth factors, cytokines, chemokines, monoclonal antibodies.

3. Use according to claim 2, wherein the hematopoietic growth factor group comprises thrombopoietin (TPO), the cytokines group comprises IL-1, IL-3, IL-6, IL-11, insulin-like growth factor 1 (IGF-1), G-CSF, GM-CSF or SCF; the chemokines group comprises MIP-1α, MPIF-1, MPIF-2 or EU-2; the monoclonal antibodies group comprises anti-VLA-4 antibodies.

4. Use according to any one of the preceding claims, wherein the medicament further comprises G-CSF.

5. Use according to any one of the preceding claims, wherein the medicament comprises growth hormone and G-CSF.

6. Use according to any one of the preceding claims, wherein the CD34 negative pluripotent peripheral blood cells are long-term culture-initiating cells (LTC-IC).

7. Use according to any one of the preceding claims, for treating a neoplastic disease, leukemia, lymphoma, a hematological disorder, malignancies, congenitally or genetically determined hematopoietic abnormalities, anemia, plastic anemia, neutropenia and/or osteopetrosis.

8. Use according to claim 7, wherein the lymphoma is Hodgkin's lymphoma.

9. Use according to any one of the preceding claims, wherein the increase is mediated by the mobilization of CD34 negative pluripotent peripheral blood cells capable of regenerating hematopoiesis in the patient from the bone marrow.

10. Use according to any one of the preceding claims, wherein the number of CD34 negative pluripotent peripheral blood cells capable of regenerating hematopoiesis in a human being is at least about 50, 100, 500 or 1000 cells per milliliter of blood.

11. Use according to any one of the preceding claims, wherein the growth hormone is administered in an amount of 10 to 500 µg per kg per administration.

12. Use according to any one of the preceding claims wherein the growth hormone is administered in an amount of around 100 µg per kg per administration.

13. Use according to any one of claims 4 to 12, wherein G-CSF is administered in an amount of 1 to 100 µg per kg per day.

14. Use according to claim 13, wherein G-CSF is administered in an amount of around 5 to 10 µg per kg per day.

15. Use according to any one of the preceding claims, wherein the administration is made by parenteral, subcutaneous, intravenous, intramuscular, intraperitoneal, transdermal or oral routes.

16. Use according to claim 15, wherein the administration is made subcutaneously.

17. Use according to any one of the preceding claims, wherein the administration is daily or three times a day.

18. Use according to any one of claims 4 to 17, wherein the administration of growth hormone is made three times a day and the administration of G-CSF is daily.

19. Use according to any one of the preceding claims, wherein the administration is made over a period of about 1 to 14 days or, until leukapheresis, until mobilization or peripheralization of circulating cells capable of regenerating hematopoiesis in vivo, until increase of the number of circulating cells capable of regenerating hematopoiesis in vivo or until engraftment.

20. Use according to any one of the preceding claims, wherein the administration(s) is/are made before or after chemotherapy, radiotherapy, myelotoxic or myelosuppressive therapy, transplantation of cells capable of regenerating hematopoiesis in vivo or bone marrow transplantation.

21. Use according to claim 20, wherein the administration(s) begin(s) around seven days after the beginning of a chemotherapeutic treatment or around 2 days after the end of a chemotherapeutic treatment.

22. Use according to claim 21, wherein the administration(s) is/are made after chemotherapeutic treatment with ifosphamide and/or vinorelbine.

23. Use according to any one of the preceding claims, wherein the CD34 negative pluripotent peripheral blood cells are retrieved by leukapheresis.

24. Use according to any one of the preceding claims, wherein growth hormone is human growth hormone.

25. Use according to any one of the preceding claims, wherein growth hormone is recombinant growth hormone.

26. Use according to any one of the preceding claims, wherein G-CSF is recombinant G-CSF.

## Patentansprüche

1. Verwendung von Wachstumshormon oder einem seiner Abkömmlinge zur Herstellung eines Medikaments zur Erhöhung, in einem Patienten, der der Blutneubildung bedarf, der Anzahl CD34-negativer, pluripotenter, periphärer Blutzellen, die zur Blutneubildung geeignet sind, so dass besagte CD34-negative, pluripotente, periphäre Blutzellen in einer Menge bereitgestellt werden, die zur autologen Transplantation ausreicht.

2. Verwendung nach Anspruch 1, wobei das Medikament ferner eine oder mehrere Verbindungen(en) ausgewählt aus den folgenden Gruppen von Verbindungen umfasst: hämatopoetische Wachstumsfaktoren, Zytokine, Chemokine, monoklonale Antikörper.

3. Verwendung nach Anspruch 2, wobei die hämatopoetische Wachstumsfaktorgruppe Thrombopoietin (TPO) umfasst, die Gruppe der Zytokine IL-1, IL-3, IL-6, IL-11, Insulin-ähnlichen Wachstumsfaktor 1 (IGF-1), G-CSF, GM-CSF oder SCF umfasst; die Gruppe der Chemokine MIP-1α, MPIF-1, MPIF-2 oder EU-2 umfasst; die Gruppe der monoklonalen Antikörper anti-VLA-4-Antikörper umfasst.

4. Verwendung gemäß einem der vorhergehenden Ansprüche, wobei das Medikament ferner G-CSF umfasst.

5. Verwendung nach einem der vorhergehenden Ansprüche, wobei das Medikament Wachstumshormon und G-CSF umfasst.

6. Verwendung nach einem der vorhergehenden Ansprüche, wobei die CD34-negativen, pluripotenten, periphären Blutzellen Langzeitkulturinitiierende Zellen (LTC-IC) sind.

7. Verwendung nach einem der vorhergehenden Ansprüche zur Behandlung einer neoplastischen Erkrankung, von Leukämie, eines Lymphoms, einer hämatologischen Störung, von bösartigen Tumoren, von erblich bedingten oder genetisch festgestellten hämatopoetischen Abnormalitäten, einer Anämie, einer plastischen Anämie, einer Neutropenie und/oder von Osteopetrose.

8. Verwendung nach Anspruch 7, wobei das Lymphom ein Hodgkin-Lymphom ist.

9. Verwendung nach einem der vorhergehenden Ansprüche, wobei die Erhöhung durch die Mobilisierung CD34-negativer, pluripotenter, periphärer Blutzellen, die zur Blutneubildung geeignet sind, im Patienten aus dem Knochenmark herbeigeführt wird.

10. Verwendung nach einem der vorhergehenden Ansprüche, wobei die Anzahl CD34-negativer, pluripotenter, periphärer Blutzellen, die zur Blutneubildung in einem Menschen geeignet sind, mindestens etwa 50, 100, 500 oder 1000 Zellen pro ml Blut beträgt.

11. Verwendung nach einem der vorhergehenden Ansprüche, wobei das Wachstumshormon in einer Menge von 10 bis 500 µg pro kg pro Verabreichung verabreicht wird.

12. Verwendung nach einem der vorhergehenden Ansprüche, wobei das Wachstumshormon in einer Menge von ungefähr 100 µg pro kg pro Verabreichung verabreicht wird.

13. Verwendung nach einem der Ansprüche 4 bis 12, wobei G-CSF in einer Menge von 1 bis 100 µg pro kg pro Tag verabreicht wird.

14. Verwendung nach Anspruch 13, wobei G-CSF in einer Menge von ungefähr 5 bis 10 µg pro kg pro Tag verabreicht wird.

15. Verwendung nach einem der vorhergehenden Ansprüche, wobei die Verabreichung über parenterale, subcutane, intravenöse, intramuskuläre, intraperitoneale, transdermale oder orale Wege durchgeführt wird.

16. Verwendung nach Anspruch 15, wobei die Verabreichung subcutan durchgeführt wird.

17. Verwendung nach einem der vorhergehenden Ansprüche, wobei die Verabreichung täglich oder dreimal täglich stattfindet.

18. Verwendung nach einem der Ansprüche 4 bis 17, wobei die Verabreichung von Wachstumshormon dreimal täglich durchgeführt wird und die Verabreichung von G-CSF täglich.

19. Verwendung nach einem der vorhergehenden Ansprüche, wobei die Verabreichung über eine Dauer von etwa 1 bis 14 Tagen, bis zur Laukapherese, bis zur Mobilisierung oder Periphärisierung von zirkulierenden Zellen, die zur *in vivo* Blutneubildung geeignet sind, bis zur Erhöhung der Anzahl zirkulierender Zellen, die zur *in vivo* Blutneubildung geeignet sind, oder bis zum Anwachsen durchgeführt wird.

20. Verwendung nach einem der vorhergehenden Ansprüche, wobei die Verabreichung(en) vor oder nach einer Chemotherapie, einer Strahlentherapie, einer myelotoxischen oder myelosuppressiven Therapie, einer Transplantation von Zellen, die zur *in vivo* Blutneubildung geeignet sind oder einer Knochenmarkstransplantation durchgeführt wird/werden.

21. Verwendung nach Anspruch 20, wobei die Verabreichung(en) ungefähr sieben Tage nach dem Beginn einer chemotherapeutischen Behandlung oder ungefähr 2 Tage nach dem Ende einer chemotherapeutischen Behandlung beginnt/beginnen.

22. Verwendung nach Anspruch 21, wobei die Verabreichung(en) nach einer chemotherapeutischen Behandlung mit Ifosphamid und/oder Vinorelbin durchgeführt wird/werden.

23. Verwendung nach einem der vorhergehenden Ansprüche, wobei die CD34-negativen, pluripotenten, periphären Blutzellen mittels Leukapherese zurückgewonnen werden.

24. Verwendung nach einem der vorhergehenden Ansprüche, wobei das Wachstumshormon menschliches Wachstumshormon ist.

25. Verwendung nach einem der vorhergehenden Ansprüche, wobei das Wachstumshormon rekombinantes Wachstumshormon ist.

26. Verwendung nach einem der vorhergehenden Ansprüche, wobei G-CSF rekombinantes G-CSF ist.

## Revendications

1. Utilisation d'une hormone de croissance ou de l'un de ses dérivés en vue de la fabrication d'un médicament conçu pour faire augmenter, chez un patient nécessitant une régénération de l'hématopoïèse, le nombre de cellules pluripotentes CD34-négatives du sang périphérique, de manière à ce que ces cellules pluripotentes CD34-négatives du sang périphérique se trouvent en une quantité suffisante pour une transplantation autologue.

2. Utilisation conforme à la revendication 1, dans laquelle le médicament comprend en outre un ou plusieurs composé(s) choisi(s) dans les groupes de composés suivants : facteurs de croissance hématopoïétiques, cytokines, chimiokines, et anticorps monoclonaux.

3. Utilisation conforme à la revendication 2, dans laquelle le groupe des facteurs de croissance hématopoïétiques comprend la thrombopoïétine ou TPO, le groupe des cytokines comprend les interleukines IL-1, IL-3, IL-6, IL-11 et les facteurs IGF-1 (facteur de croissance 1 apparenté à l'insuline), G-CSF, GM-CSF et SCF, le groupe des chimiokines comprend les facteurs MIP-la, MPIF-1, MPIF-2 et EU-2, et le groupe des anticorps monoclonaux comprend les anticorps anti-VLA-4.

4. Utilisation conforme à l'une des revendications précédentes, dans laquelle le médicament comprend en outre un facteur G-CSF.

5. Utilisation conforme à l'une des revendications précédentes, dans laquelle le médicament comprend une hormone de croissance et un facteur G-CSF.

6. Utilisation conforme à l'une des revendications précédentes, dans laquelle les cellules pluripotentes CD34-négatives du sang périphérique sont des cellules LTC-IC (cellules initiatrices de colonies à long terme).

7. Utilisation conforme à l'une des revendications précédentes, en vue de traiter une maladie néoplasique, une leucémie, un lymphome, un trouble hématologique, des affections malignes, des anomalies hématopoïétiques congénitales ou d'origine génétique, une anémie, une anémie aplasique, une neutropénie et/ou une ostéopétrose.

8. Utilisation conforme à la revendication 7, dans laquelle le lymphome est un lymphome de Hodgkin.

9. Utilisation conforme à l'une des revendications précédentes, dans laquelle ladite augmentation est médiée par la mobilisation des cellules pluripotentes CD34-négatives du sang périphérique capables de régénérer, chez le patient, l'hématopoïèse au niveau de la moelle osseuse.

10. Utilisation conforme à l'une des revendications précédentes, dans laquelle le nombre des cellules pluripotentes CD34-négatives du sang périphérique capables de régénérer l'hématopoïèse chez un être humain vaut au moins environ 50, 100, 500 ou 1000 cellules par millilitre de sang.

11. Utilisation conforme à l'une des revendications précédentes, dans laquelle l'hormone de croissance est administrée en une quantité de 10 à 500 µg/kg par administration.

12. Utilisation conforme à l'une des revendications précédentes, dans laquelle l'hormone de croissance est administrée en une quantité d'à peu près 100 µg/kg par administration.

13. Utilisation conforme à l'une des revendications 4 à 12, dans laquelle le facteur G-CSF est administré en une quantité de 1 à 100 µg/kg par jour.

14. Utilisation conforme à la revendication 13, dans laquelle le facteur G-CSF est administré en une quantité d'à peu près 5 à 10 µg/kg par jour.

15. Utilisation conforme à l'une des revendications précédentes, dans laquelle l'administration s'effectue par voie parentérale, sous-cutanée, intraveineuse, intramusculaire, intrapéritonéale, transdermique ou orale.

16. Utilisation conforme à la revendication 13, dans laquelle l'administration s'effectue par voie sous-cutanée.

17. Utilisation conforme à l'une des revendications précédentes, dans laquelle l'administration est quotidienne ou est effectuée trois fois par jour.

18. Utilisation conforme à l'une des revendications 4 à 17, dans laquelle l'administration de l'hormone de croissance est effectuée trois fois par jour et l'administration du facteur G-CSF est quotidienne.

19. Utilisation conforme à l'une des revendications précédentes, dans laquelle l'administration est effectuée sur une période d'à peu près 1 à 14 jours, ou bien jusqu'à une leucophérèse, jusqu'à une mobilisation ou une périphéralisation de cellules circulantes capables de régénérer l'hématopoïèse *in vivo*, jusqu'à une augmentation des cellules circulantes capables de régénérer l'hématopoïèse *in vivo*, ou jusqu'à une greffe.

20. Utilisation conforme à l'une des revendications précédentes, dans laquelle la ou les administration(s) est ou sont effectuée(s) avant ou après une chimiothérapie, une radiothérapie, une thérapie myélotoxique ou myélodépressive, une transplantation de cellules capables de régénérer l'hématopoïèse *in vivo,* ou une transplantation de moelle osseuse.

21. Utilisation conforme à la revendication 20, dans laquelle la ou les administration(s) commence(nt) à peu près 7 jours après le début d'un traitement chimiothérapeutique ou à peu près 2 jours après la fin d'un traitement chimiothérapeutique.

22. Utilisation conforme à la revendication 21, dans laquelle la ou les administration(s) est ou sont effectuée(s) après un traitement chimiothérapeutique par ifosphamide et/ou vinorelbine.

23. Utilisation conforme à l'une des revendications précédentes, dans laquelle les cellules pluripotentes CD34-négatives du sang périphérique sont récupérées par leucophérèse.

24. Utilisation conforme à l'une des revendications précédentes, dans laquelle l'hormone de croissance est de l'hormone de croissance humaine.

25. Utilisation conforme à l'une des revendications précédentes, dans laquelle l'hormone de croissance est une hormone de croissance recombinante.

26. Utilisation conforme à l'une des revendications précédentes, dans laquelle le facteur G-CSF est un facteur G-CSF recombinant.
